Europäisches Patentamt

European Patent Office

Office européen des brevets

(19)

(11) **EP 1 512 393 A1**

(12) **EUROPÄISCHE PATENTANMELDUNG**

(43) Veröffentlichungstag:
09.03.2005 Patentblatt 2005/10

(51) Int Cl.⁷: **A61K 9/127**

(21) Anmeldenummer: 03019663.8

(22) Anmeldetag: 08.09.2003

(84) Benannte Vertragsstaaten:
**AT BE BG CH CY CZ DE DK EE ES FI FR GB GR
HU IE IT LI LU MC NL PT RO SE SI SK TR**
Benannte Erstreckungsstaaten:
**AL LT LV MK**

(71) Anmelder: **BOEHRINGER INGELHEIM PHARMA
GMBH & CO. KG
55216 Ingelheim am Rhein (DE)**

(72) Erfinder: **Die Erfindernennung liegt noch nicht
vor**

(54) **Verfahren zur Herstellung von homogenen Liposomen und Lipoplexen**

(57)    Die vorliegende Erfindung betrifft ein kontinuierliches Niederdruckextrusionsverfahren zur Herstellung von Liposomen, Verfahren zur Herstellung von Komplexen bestehend aus entsprechend hergestellten Liposomen und Nukleinsäuremolekülen (Lipoplexen), Verfahren zur stabilen Lagerung entsprechender Lipoplexe, sowie entsprechend hergestellte Liposomen und Lipoplexen.

EP 1 512 393 A1

**Beschreibung**

**Technisches Gebiet**

Die vorliegende Erfindung betrifft Verfahren zur Herstellung von Liposomen und Komplexen bestehend aus Liposomen und Nukleinsäuremolekülen (Lipoplexen), Verfahren zur stabilen Lagerung entsprechender Lipoplexe, sowie entsprechend hergestellte Liposomen und Lipoplexe.

**Hintergrund der Erfindung**

Mit Aufkommen gentherapeutischer Behandlungsmethoden wurden Liposomen als eine viel versprechende Alternative zu viralen Gentransfersystemen erkannt. Das Komplexieren von Nukleinsäuren in/mit Liposomen und die Verwendung entsprechender Komplexe (Lipoplexe) für gentherapeutische Ansätze stellt die Liposomentechnologie jedoch vor neue Herausforderungen. Für einen effizienten und reproduzierbaren Gentransfer müssen eine Vielzahl an chemischen und physikalischen Parameter der Liposomen / Lipoplexe definiert werden. Darüber hinaus muss das Verfahren unter aseptischen Bedingungen durchführbar sein und den strengen Herstellvorschriften für Arzneimittel genügen.

Die Entwicklung kationischer Liposomen stellt einen wesentlichen Schritt bei der Bereitstellung von nicht viralen gentherapeutisch effektiven Transfersystemen dar. Kationische Liposomen werden entweder aus einem einzelnen kationischen Lipid oder, häufiger, aus einer Kombination eines kationischen Lipids mit einem neutralen Amphiphil (Helferlipid, Co-Lipid) hergestellt. Das erste Reagenz dieser Art, DOTMA ([N-1-(2,3-dioleyloxy)propyl]-N,N,N,-triemthylammoniumchlorid), ist nach Mischung mit einer äquimolaren Menge an DOPE (Dioleoylphosphatidylethanolamin) in der Lage Säugerzellen in vitro und in vivo zu transfizieren (Felgner et al. (1989) Nature 337, 387-388). Mittlerweile sind eine Vielzahl an kationischen Lipiden bekannt, die entweder direkt oder in Verbindung mit neutralen Amphiphilen beim Gentransfer verwendet werden. Dazu zählen z.B. DORI (1,2-Dioleoyloxycarbonylpropyl-3-dimethylhydroxyethylammoniumbromid, DORIE (1,2-Dioleyloxypropyl-3-dimethylhydroxyethylammoniumbromid), DOTAP (Dioleoyltrimethylammoniumpropan(N-[1-(2,3-Dioleoyloxy)propyl]-N,N,N-trimethylammonium-methylsulfat)), DMRIE (N-(1,2-Dimyristoyloxypropyl)-N,N-dimethyl-N-hydroxyethylammonium-bromid)), DOGS (Dioctadecylamidologycylspermine), DOSPA (2,3-dioleyloxy-N-[2(spermin-carboxamido) ethyl]-N,N-diemthyl-1-propanaminiumtrifluoroacetat), PDMAEMA (Poly (2-dimethylamino)ethyl-methacrylat), DDAB (Dimethyldioctadecylammonium) DC-Chol 3β-[N-(N',N'-Dimethylaminoethyl)carbamoyl]-cholesterin) und DAC-Chol ((3-beta[N(N,N'-Dimethylamino-ethan)carbamoyl]-cholesterin)). Ferner zählen hierzu verschiedene Spermincholesteryl-carbamate, wie beispielhaft in WO96/18372 oder 1,4-Dihydropyridine-Derivative, wie beispielsweise in WO01/62946 beschrieben. Eine nicht abschließende Zusammenfassung relevanter kationischer Lipide findet sich beispielsweise auch in der Publikation von Miller (1998), Angew. Chem. 110, 1862-1880, auf die hier ausdrücklich inhaltlich Bezug genommen wird. Einige kationische Lipide und Gemische sind auch kommerziell erhältlich wie Effectene™ und SuperFect™ (Qiagen, Hilden, Deutschland), FuGene 6™ (Roche, Mannheim, Deutschland), LipoFectin™, LipoFectin2000™, LipoFECTAMINE Plus™ (Invitrogen, Karlsruhe, Deutschland).

Seit Ende der 80iger Jahre wurde die Lipofektion, also die Transfektion von in/mitLiposomen komplexierter Nukleinsäure, vorangetrieben und erfolgreich an vielen Zelltypen und Zelllinien ausprobiert. Für humane gentherapeutische Applikationen hat sich gezeigt, dass die Verwendung von Lipoplexen eine viel versprechende Methode darstellt. (Galanis (2002) Current Opinion Molec. Therapeutics 4: 80-87; Stopeck et al. (2001) Clinical Cancer Res. 7: 2285-2291.; Voges et al. 2002) Human Gene Therapy. 13: 675-685; Jacobs et al. (2001) Lancet. 358: 727-729; Morgtan (ed.) Gene Therapy Protocols (2002) Humana Press Inc. New Jersey).

Für humane Applikationen benötigt man eine einfache und praktische Darreichungsform. Die Herausforderung besteht insbesondere in der Herstellung von physikalisch und chemisch stabilen Lipoplexen und der Etablierung eines Verfahrens zur reproduzierbaren Herstellung dieser Komplexe mit einer gleich bleibenden Qualität in den biophysikalischen und biologischen Eigenschaften.

Das Zusammenbringen von kationisch geladenen Amphiphilen wie z.B. Lipiden und negativ geladenen Nukleinsäuremolekülen führt über elektrostatische Wechselwirkungen zur Bildung von Komplexen. Je nach Mischvorgang, Konzentration der Ausgangsstoffe, Formulierung usw. kann zu einer Ausflockung der Komplexe kommen (Gershon et al. (1993) Biochemistry 32: 7143-7151; Lasic et al. (1997) J. Am. Chem. Soc. 119: 832-833; Eastman et al. (1997) BBA 1325: 41-62). Die Dispersion ist somit nicht stabil und das Produkt untauglich für klinische Anwendungen, da die dabei entstehenden Aggregate sehr groß werden (mehrere Mikro- bis Millimeter). Da es sehr schwierig ist Lipoplexe im Hinblick auf ihr Aggregationsverhalten zu stabilisieren, werden die Lipoplexe für klinische Anwendungen frisch vom Arzt im Krankenhaus ("bed-side") hergestellt oder gefroren gelagert (Galanis (2002) supra; Stopeck et al. (2001) supra; Voges et al. (2002) supra; Jacobs et al. (2001) supra; Morgtan (2002) supra, Gao & Huang (1995) Gene Therapy 2: 710-722; Felgner et al. (1995) supra; Nabel et al. (1994) Hum. Gene Therapy 5: 57-77 und 1089-1094).

Deshalb ist es sehr wesentlich Verfahren zu entwickeln, die eine reproduzierbare Lipoplexqualität liefern.

Die biophysikalischen Eigenschaften der Lipoplexe hängt unter anderem von den Eigenschaften und der Qualität der Liposomen ab, die zur Komplexierung von Nukleinsäure verwendet werden. In der Literatur sind verschiedene Methoden zur Herstellung von liposomalen Suspensionen und Liposomenpräparaten beschrieben. So lassen sich beispielsweise Liposomen durch Beschallung von Lipid-haltigen Lösungen mittels eines Ultraschallbades oder eines Schwingstabes herstellen. Die entsprechenden Verfahren stellen stark energieverbrauchende Methoden dar (Perrett et al., (1991) J. Pharmacy & Pharmacology, 43: 154-161), die sich nur schwer auf einen pharmazeutischen Herstellungsmaßstab vergrößern (= upscalen) lassen. Des weiteren besteht die Gefahr, dass durch die Verwendung eines Schwingstabes die Liposomen durch kleine Metallpartikel verunreinigen. Die Produktqualität (Größe der Liposomen) ist nur schwer zu reproduzieren und die dabei gebildeten Liposomen sind zumeist sehr klein ($\leq$ 250 nm). Gregoriadis et al. (1990) Int. J. Pharmaceutics 65: 235-242 beschreiben, dass Liposomen über eine Dehydratations-Hydratations Methode gebildet werden können. Dabei können auch Wirkstoffe in die Liposomen eingeschlossen werden. Die gebildeten Lipidsuspensionen zeigen allerdings eine hohe Inhomogenität bezüglich Größenverteilung und Polydispersität. Homogenere Lipidsuspensionen erhält man erst nach einem zusätzlichen Prozessschritt, der Mikrofluidisierung (Washington & Davis, (1988) Int. J. Pharmaceutics 44: 169-176; Vuillemard JC. (1991) J. Microencapsulation 8: 547-562). Diese Methode arbeitet allerdings mit sehr hohen Drücken.

Die Herstellung von Liposomen mittels Extrusionsverfahren ist für die Präparation von Phospholipid-Dispersionen bekannt (Elorza et al. (1993) J. Microencapsulation 10: 237-248; Berger et al. (2001) Int. J. Pharmaceutics 223: 55-68). Hierbei wird zumeist mit sehr hohen Drücken gearbeitet, insbesondere wenn die Extrusion durch Membranen mit einer Porengröße von kleiner 1000 nm erfolgt (im Fall von Berger et al. (*supra*) mit Arbeitsdrücken von über 50 x $10^5$ Pa). Dies führt zum einen zu kleinen Liposomen und ist zum anderen mit einem erheblichen Aufwand bei der Umsetzung in ein großtechnisches Verfahren verbunden. Oft wird dieses Extrusionsverfahren auch mit einem weiteren Prozessschritt kombiniert, beispielsweise einem Gefrier-Tau-Schritt, um die Produktqualität zu erhöhen (Mayer et al. 1986) BBA 858: 161-168; Hope et al. (1985) BBA 812: 55-65; Nayar et al. (1989) BBA 986: 200-206) beschreiben eine Extrusionsmethode von Gelphasen-Lipiden, die allerdings ebenfalls sehr hohe Drücke zwischen 17,5 bis 49 x $10^5$ Pa erfordert. Je nach verwendetem Lipid und Extrusionsdruck beschreiben die Autoren vorwiegend die Herstellung von Liposomen mit sehr kleinen Durchmessern, zumeist unterhalb von 200 - 150 nm.

Ebenfalls mit hohen Drücken arbeiten Sorgi & Huang (1996) Int. J. Pharmaceutics 144: 131-139, die die Herstellung von kationischen Liposomen mit Hilfe des Mikrofluidiser beschreiben. Der verwendete Arbeitsdruck lag bei ungefähr 6.2 x $10^5$ Pa. Der Durchmesser der hierbei erzeugten Liposomen lag unterhalb von 200 nm (vgl. auch W096/27393). Die Patentanmeldung WO98/17814 beschreibt darüber hinaus kationische Liposomen mit einer Größe von ungefähr 800 nm.

Zusammenfassend lässt sich festhalten, dass die im Stand der Technik bekannten Verfahren entweder zu sehr kleinen Liposomen (< 200 nm) führen, die für den Transfer von Nukleinsäuren aufgrund ihrer geringen Transfektionseffizienz nur sehr begrenzt einsetzbar sind, oder zu inhomogenen Liposomen, die zwar ausreichend transformierbar sind, aber nicht über längere Zeiten stabil sind und den strengen Qualitätsanforderungen für Arzneimittel nicht genügen können.

Eine Aufgabe zur vorliegenden Erfindung bestand folglich darin, ein Verfahren zur Herstellung von homogenen Liposomen zur Verfügung zu stellen, die eine hohe Lagerstabilität aufweisen, und die Herstellung von homogenen Lipoplexen erlauben, die über eine hinreichende Transfektionseffizienz bei guter Stabilität verfügen. Eine weitere Aufgabe bestand darin entsprechende Liposomen mit einer Größe von 250 - 800 nm und Lipoplexe mit einer Größe von 250 - 600 nm bereitzustellen.

Eine weitere Aufgabe zur vorliegenden Erfindung bestand darin, ein entsprechendes Verfahren zur Liposomen- bzw. Lipoplexherstellung zu finden, bei dem sich Liposomen bzw. Lipoplexe unter GMP-Bedingungen herstellen lassen. Dies bedeutet die Herstellung von reproduzierbar homogenen Liposomen-/ Lipoplex-Chargen unter aseptischen Bedingungen in einem größeren Maßstab.

Eine weitere Aufgabe zur vorliegenden Erfindung bestand darin, entsprechende homogene und lagerstabile Liposomen und Lipoplexe bereitzustellen, die über eine hinreichende Transfektionseffizienz bei guter Stabilität und GMP-Qualität verfügen.

## Zusammenfassung der Erfindung

Die vorliegende Erfindung betrifft ein Verfahren zur Herstellung von homogenen Liposomen, bei dem eine Lipidsuspension in einem kontinuierlichen Verfahren durch eine poröse Membran, vorzugsweise mit einer Porengröße zwischen 600 - 900 nm unter Niederdruckbedingungen bei kleiner 3 x $10^5$ Pa extrudiert wird. Es hat sich gezeigt, dass ein entsprechendes Verfahren bei der Verwendung von kationischen Liposomen, bzw. von Liposomen enthaltend ein kationisches Lipid und ein neutrales Amphiphil (z.B. bestehend aus DC-Chol/DOPE oder DAC-Chol/DOPE) zu stabilen und homogenen Liposomenmischungen mit Liposomen mit einer Größe von 250 - 800 nm, vorzugsweise von 250 - 600 nm und einem Polydispersitätsindex von ≤ 0.6, vorzugsweise ≤ 0.5, weiter bevorzugt von ≤ 0.4 führen. Besonders vorteilhaft hat sich die Verwendung einer Polycarbonat-Membran als Extrusionsmembran erwiesen.

Nach einer bevorzugten Ausführungsform beträgt die Konzentration der Liposomen in der Lipidsuspension zwischen 0.04 und 5 mg/ml, vorzugsweise zwischen 0.1 - 2 mg/ml, insbesondere zwischen 0.1 - 1 mg/ml. Als besonders geeignet haben sich in dem Zusammenhang Flussraten zwischen 10 - 250 ml/min, vorzugsweise zwischen 50 - 150 ml/min, besonders bevorzugt zwischen 75 - 120 ml/min erwiesen. Das erfindungsgemäße Extrusionsverfahren lässt sich auch bei Raumtemperatur durchführen, ohne dass die Qualität der Liposomen beeinträchtigt wird.

Nach einer weiteren Ausführungsform wird das entsprechende erfindungsgemäße Verfahren in einem geschlossenen System unter aseptischen Bedingungen durchgeführt. Die Liposomen bzw. die Liposomensuspension kann zuvor durch Membranen mit einer Porengröße bis 1000 nm vorfiltriert werden.

Nach einer weiteren Ausführungsform betrifft die vorliegende Erfindung ein Verfahren zur kontinuierlichen Niederdruckextrusion von Liposomen, vorzugsweise von Liposomen die ein kationisches Lipid oder eine Mischung aus einem kationischen Lipid und einem neutralen Amphiphil enthalten, vorzugsweise eine Kombination aus DC-Chol oder DAC-Chol und DOPE, bei einem Druck unterhalb von $3x10^5$ Pa, einer Flussrate zwischen 10 - 250 ml/min und einer Lipidkonzentration zwischen 0.04 - 5 mg/ml dadurch gekennzeichnet, dass die Lipidsuspension kontinuierlich zwischen 2 - 20 mal durch die poröse Membran extrudiert wird. Dieses Verfahren führte überraschenderweise zu besonders homogenen Liposomen mit einer Größe von 250 - 600 nm, vorzugsweise von 280 - 500 nm, besonders bevorzugt von 280 - 400 nm, und einem Polydispersitätsindex der bei ≤ 0.5 vorzugsweise bei ≤ 0.4 lag.

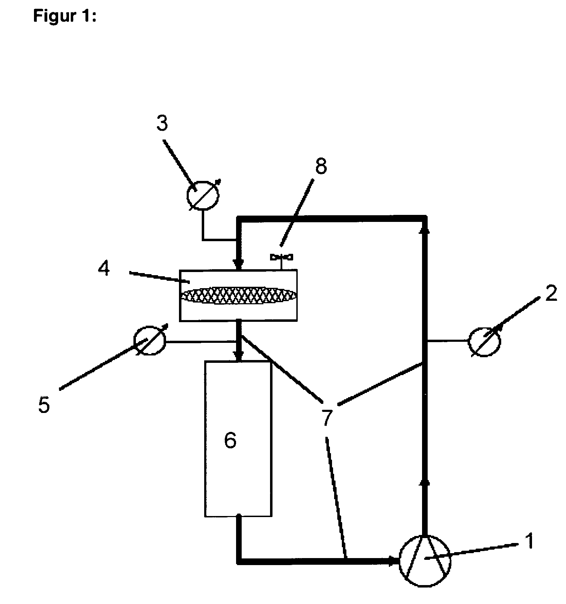

Als besonders geeignet zur großtechnischen Herstellung von entsprechenden erfindungsgemäßen Liposomen hat sich die Verwendung einer Apparatur wie in Figur 1 dargestellt erwiesen. Die Apparatur besteht aus einer mechanischen oder elektrischen Pumpe (1), einer Durchflussmessungsregelung (2), einem Manometer zur Messung des Filtrationsdrucks (3), einem Filterhalter mit einer Extrusionsmembran (Porengröße z.B. 600 nm / Ø z.B. 47 mm) (4) und Belüftungsventil (8), eine Temperaturmessvorrichtung (5), einem Vorlagegefäß (6) und einem ringförmigen Leitungssystem (7), welches eine kontinuierliche gerichtete Durchströmung der Extrusionsmembran und den Rückfluss in das Vorlagegefäß ermöglicht.

Die vorliegende Erfindung betrifft ferner auch Liposomen bzw. Liposomenmischungen die Liposomen enthalten, die nach einem der hier beschriebenen erfindungsgemäßen Verfahren hergestellt werden. Die vorliegende Erfindung betrifft insbesondere Liposomenmischungen bestehend aus Liposomen mit einer definierten Größe zwischen 250 und 800 nm, vorzugsweise zwischen 250 und 600 nm, wobei die Liposomen ein kationisches Lipid und eine neutrales Amphiphil enthalten und dadurch gekennzeichnet sind, dass der Polydispersitätsindex der Liposomenmischung einen Wert ≤ 0.60, vorzugsweise ≤ 0.50, besonders bevorzugt ≤ 0.4 aufweist. Nach einer weiteren Ausführungsform ist die Liposomenmischung dadurch gekennzeichnet, dass es sich bei dem kationischen Lipid um ein Cholesterol wie beispielsweise DC-Chol ((3-beta[N(N',N'-Dimethylaminoethan)carbamoyl]-cholesterin)) oder um DAC-Chol ((3-beta[N(N,N'-Dimethylamino-ethan)carbamoyl]-cholesterin)) handelt. Nach einer weiter bevorzugten Ausführungsform enthalten die erfindungsgemäßen Liposomen ein Ethanolaminderivat, beispielsweise Dioleoylphosphatidylethanolamin (DOPE)).

Darüber hinaus betrifft die vorliegende Erfindung ein Verfahren zur Mischung von erfindungsgemäßen Liposomen mit Nukleinsäuremolekülen (Nukleinsäuremolekül = Nukleinsäuren). Als besonders vorteilhaft hat sich die Mischung über ein sog. Y-Stück erwiesen, welches ein gleichmäßiges und kontinuierliches Zusammenführen der Liposomen und Nukleinsäuremoleküle erlaubt. Des weiteren hat sich herausgestellt, dass ein Liposomen-Nukleinsäure-Ladungsverhältnis +/- zwischen 4 - 0.01, vorzugsweise zwischen +/- 1.25 - 0.75 zu besonders stabilen und homogenen Lipoplexen führt. Als besonders vorteilhaft hat sich das Zusammenführen von gleichen Volumina an einer Liposomen enthaltenden Suspension und der Nukleinsäure enthaltenden Lösung erwiesen.

Nach einer weiteren Ausführungsform beträgt die Konzentration der Liposomen bei der Mischung von Liposomen und Nukleinsäure zwischen 0.02 - 1 mg/ml. Als vorteilhaft haben sich Flussraten zwischen 100 - 500 ml/min bei der Mischung von Liposomen und Nukleinsäuren herausgestellt. Das entsprechende Verfahren lässt sich in einer geschlossenen Apparatur durchführen, so dass die Herstellung der Lipoplexe unter aseptischen Bedingungen möglich ist.

Das entsprechende erfindungsgemäße Verfahren ermöglicht die Herstellung einer Lipoplexmischung bestehend aus Lipoplexen mit einer definierten Größe zwischen 250 - 600 nm, vorzugsweise zwischen 275 - 500 nm, besonders bevorzugt zwischen 275 - 400 nm, und mit einem Polydispersitätsindex von ≤ 0.50, vorzugsweise von ≤ 0.40. Nach einer weiteren Ausführungsform betrifft die vorliegende Erfindung folglich Lipoplexe, die nach dem hier beschriebenen erfindungsgemäßen Verfahren hergestellt werden, insbesondere solche, mit einer definierten Größe zwischen 250 - 600 nm, vorzugsweise zwischen 275 - 500 nm, besonders bevorzugt zwischen 275 - 400 nm, und mit einem Polydispersitätsindex von ≤ 0.50, vorzugsweise von ≤ 0.40.

In einer weiteren Ausführungsform betrifft die vorliegende Erfindung ein Verfahren zur Langzeitlagerung von entsprechend hergestellten Lipoplexen, beispielsweise durch Lyophilisation in Gegenwart eines geeigneten Stabilisators enthaltend die Schritte **(a)** Einfrieren der Lipoplexmischung auf eine Temperatur von ≤ - 50°C; **(b)** Trocknen der Lipoplexmischung bei ungefähr - 20°C für zumindest 35 Stunden **(c)** Nachtrocknen der Lipoplexmischung bei ungefähr 20°C für zumindest 10 Stunden.

Nach einer bevorzugten Ausführungsform enthält das Verfahren zur Lyophilisation der erfindungsgemäßen Lipoplexmischung in Gegenwart eines geeigneten Stabilisators die nachfolgenden Schritte: **(a)** Einfrieren der Lipoplexmischung auf eine Temperatur von $\leq$ - 50°C mit einer Temperaturerniedrigungsrate von ungefähr $\leq$ 1°C/min; **(b)** Inkubieren der Lipoplexmischung bei $\leq$ -50°C für zumindest 2 Stunden; **(c)** Erwärmen der Lipoplexmischung auf ungefähr - 20°C mit einer Erwärmungsrate von ungefähr $\leq$ 0.3°C/min; **(d)** Trocknen der Lipoplexmischung bei ungefähr - 20°C für zumindest 35 Stunden; **(e)** Erwärmen der Lipoplexmischung von ca. - 20°C auf ca. 20°C mit einer Erwärmungsrate von ungefähr $\leq$ 0.44°C/min; und **(f)** Nachtrocknen der Lipoplexmischung bei ca. 20°C für zumindest 10 Stunden. Als besonders geeignet hat sich die Trocknung unter Punkt (d) bei einem Druck zwischen 0.01 - 0.1 mbar, vorzugsweise zwischen 0.025 - 0.05 mbar herausgestellt.

Ferner betrifft die vorliegende Erfindung auch Lipoplexlyophilisate erhältlich nach einem der hier beschriebenen erfindungsgemäßen Verfahren, sowie die Verwendung von den hier beschriebenen homogenen Lipoplexen oder Lipoplexlyophilisaten zur Herstellung von oder als Arzneimittel in der Gentherapie zur Transfektion von Säugerzellen.

## Beschreibung der Abbildungen

Figur 1: Schematischer Aufbau einer kontinuierlichen Extrusionsapparatur bestehend aus einer Schlauchpumpe (1), Durchflussmessungsregelung (2), Manometer zur Messung des Filtrationsdruck (3), Filterhalter mit Extrusionsmembran von definierter Porengröße, z.B. 600 nm $\varnothing$ 47 mm (4) und Belüftungsventil (8), Temperaturmessung (5), einem Vorlagegefäß (6) und einem ringförmigen Leitungssystem (7).

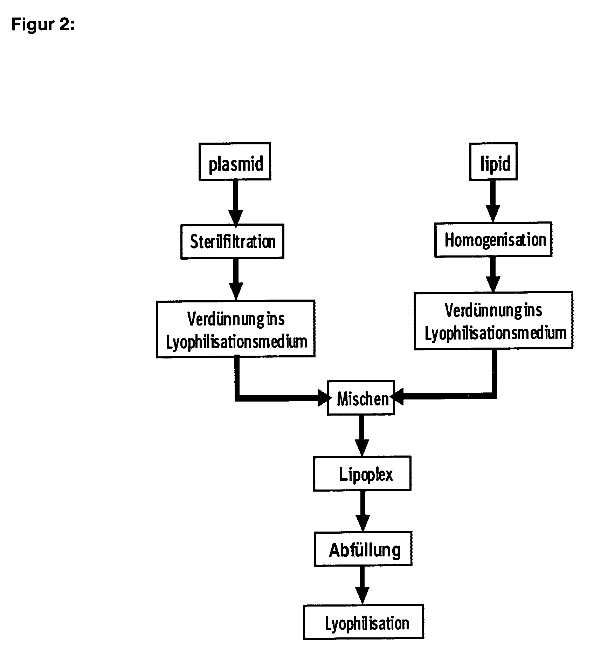

Figur 2: Fließdiagram zur Herstellung von Lipoplexen definierter Teilchengröße.

Figur 3: Schematischer Ablauf zur Herstellung von lagerfähigen Lipoplexen. Vorlagegefäß für Lipidsuspension (1), eine erste Pumpe (2), Extrusionsvorrichtung mit einer porösen Membran von 600 - 900 nm (3), Ventile (4), Verzweigung (5), Y-Stück (6), Vorlagegefäß für Nukleinsäuren (7), eine zweite Pumpe (8) und einem Auffanggefäß für Lipoplexe (9).

Figur 4: Fließbild zur Herstellung von DC30/Nukleinsäure Lipoplexen im Maßenverhältnis 4:1. Chargengröße: 3750 ml Bulk, Dosis: 0.025 mg/ml Nukleinsäure.

Figur 5: Fließbild zur Herstellung von DAC30/Nukleinsäure Lipoplexen im Maßenverhältnis 5:1. Chargengröße: 700 ml Bulk, Dosis: 0.025 mg/ml Nukleinsäure.

Figur 6: (A) REM (Raster Elektronen Mikroskopie) Aufnahme der Lyophilisationskuchenoberfläche. (B) REM (Raster Elektronen Mikroskopie) Aufnahme im Lyophilisationskuchen.

Figur 7: Einfluss der unterschiedlichen Mischungsreihenfolge bei der Lipoplexherstellung von Lipofectin und pAH7-EGFP Plasmid im Massenverhältnis von 6:1 (w/w) auf die Transfektionseffizienz und Lipoplex-(Aggregat)-Größe. LzD = Lipid zu DNA, DzL = DNA zu Lipid. Die Lipoplexgröße wurde mittels PCS bestimmt. Die Transfektionseffizienz wurde an A-10 SMC (smooth muscle cells) getestet.

Figur 8: Bioaktivität (Transfektionseffizienz) von DAC30/pMCP-1 5:1 (w/w) Lipoplexe bei 4°C über mehrere Monate gelagert. Die Transfektionseffizienz wird auf einen internen Standard bezogen.

Figur 9: Y-Stück zur Mischung von Nukleinsäure und Liposomen.

## Detaillierte Beschreibung der Erfindung

Die vorliegende Erfindung betrifft ein Verfahren zur Herstellung von homogenen Liposomen, bei dem eine Lipidsuspension in einem kontinuierlichen Verfahren durch eine poröse Membran mit einer Porengröße von vorzugsweise 600 - 900 nm extrudiert wird, dadurch gekennzeichnet, dass die Extrusion unter Niederdruckbedingungen bei Drücken kleiner $3 \times 10^5$ Pa durchgeführt wird. Die entsprechend hergestellten Liposomen zeigen eine durchschnittliche Größe von 250 - 800 nm. Der Polydispersitätsindex der Liposomenmischung beträgt $\leq$ 0.6, vorzugsweise $\leq$ 0.5, bzw. $\leq$ 0.4.

Unter dem Begriff "Liposomen" versteht man eine wässrige lipidhaltige Suspension aus mehrschichtigen oder mehrlagigen (zumindest bestehend aus einer Lipiddoppelschicht) zumeist kugelförmigen Zusammenlagerungen von Lipidmolekülen die durch mechanisches Mischen eines trockenen Lipids in Wasser entstehen.

Der "Polydispersitätsindex" (= PI) ist ein Maß für die homogene bzw. heterogen Größenverteilung der einzelnen Liposomen in einer Liposomenmischung und gibt die Breite der Teilchenverteilung in einer Mischung an. Eine genaue Definition findet sich im Kapitel "Material und Methoden". Der PI lässt sich beispielsweise nach der im Kapitel "Material und Methoden" angegebenen Methode bestimmen, die hier als Referenzmethode dient.

Der Begriff "Niederdruckbedingungen" im Sinne der Erfindung, meint Filtrationsdrücke von kleiner 3 x $10^5$ Pa, vorzugsweise von kleiner 2 x $10^5$ Pa und besonders bevorzugt von kleiner 1 x $10^5$ Pa.

Die Extrusion durch eine Membran mit definierter Porengröße ermöglicht die Herstellung von Liposomen von definierter Größe. Es hat sich gezeigt, dass nicht nur die Porengröße sondern auch weitere Parameter, wie Druck, Flussrate, Lipidkonzentration die chemisch-physikalischen Eigenschaften der extrudierten Liposomen beeinflussen. Die Extrusion bei hohen Drücken (größer 3 x $10^5$ Pa) führt zu Liposomen mit relativ kleinen Durchmessern von ungefähr kleiner 200 nm. Entsprechende Liposomen zeigen nach Mischung mit Nukleinsäuren eine sehr geringe Transfektionseffizienz, was ihre Eignung in Bereich der Gentherapie stark beschränkt. Darüber hinaus lässt sich ein Verfahren, welche sehr hohe Arbeitsdrücke benötigt, nur unter großem technischen Aufwand in einen großtechnischen Maßstab überführen.

Mit der vorliegenden Erfindung ist es gelungen, ein Verfahren für die Herstellung von homogenen Liposomen mit einer definierten Größe zwischen 250 - 800 nm, vorzugsweise zwischen 280 - 700 nm, besonders bevorzugt zwischen 280 - 600 nm zur Verfügung zu stellen. Das erfindungsgemäße Verfahren zeichnet sich durch ein hohes Maß an Reproduzierbarkeit aus, was wiederum die Herstellung von homogenen Liposomenchargen zu gentherapeutischen Zwecken ermöglicht. Unter einer "Charge" versteht man Liposomen oder Liposomenmischungen, die aus einer definierten Menge an Ausgangsmaterial während eines Arbeitsgangs / Produktionslauf hergestellt werden. Es hat sich herausgestellt, dass die hohe Qualität (= Homogenität und Stabilität) der erfindungsgemäßen Liposomen durch die gewählten Prozessparameter und die Prozessführung positiv beeinflusst wird.

Überraschenderweise wurde gefunden, dass die Extrusion einer Lipidsuspension mit einer Lipidkonzentration von 0.04 - 5 mg/ml, vorzugsweise von 0.1 - 2 mg/ml, besonders bevorzugt von 0.1 - 1 mg/ml, noch weiter bevorzugt von 0.25 - 1 mg/ml zu besonders homogenen Liposomen / Liposomenmischungen führt, wenn sie unter Niederdruckbedingungen durch eine Membran mit einer Porengröße von 600 - 900 nm durchgeführt wird. Folglich betrifft die vorliegende Erfindung auch ein Verfahren zur Herstellung von homogenen Liposomen, bei dem eine Lipidsuspension in einem kontinuierlichen Verfahren durch eine poröse Membran mit einer Porengröße von 600 - 900 nm unter Niederdruckbedingungen bei kleiner 3x$10^5$ Pa extrudiert wird, dadurch gekennzeichnet, dass die Lipidkonzentration 0.04 - 5 mg/ml, vorzugsweise 0.1 - 2 mg/ml, besonders bevorzugt 0.1 - 1 mg/ml, noch weiter bevorzugt 0.25 - 1 mg/ml beträgt.

Es zeigte sich, dass neben der Porengröße, dem Filtrationsdruck und der Lipidkonzentration auch die Flussrate einen Einfluss auf die Homogenität der Liposomen / Liposomenmischung hat. Überraschenderweise führten Flussraten zwischen 10 - 250 ml/min, vorzugsweise zwischen 50 - 150 ml/min, besonders bevorzugt zwischen 75 - 120 ml/min zu besonders homogenen Liposomen. Folglich betrifft die vorliegende Erfindung auch Verfahren zur Herstellung von homogenen Liposomen durch Niederdruckextrusion durch eine 600 - 900 nm Membran dadurch gekennzeichnet, dass die Lipidkonzentration zwischen 0.04 - 5 mg/ml, vorzugsweise zwischen 0.1 - 2 mg/ml, besonders bevorzugt zwischen 0.1 - 1 mg/ml, noch weiter bevorzugt zwischen 0.25 - 1 mg/ml und die Flussrate bei der Extrusion zwischen 10 - 250 ml/min, vorzugsweise zwischen 50 - 150 ml/min, besonders bevorzugt zwischen 75 - 120 ml/min beträgt.

Vorzugsweise handelt es sich bei der Extrusionsmembran um eine Polycarbonatmembran mit einer Porengröße von 600 - 900 nm, beispielsweise mit einer Porengröße von 600, 650, 750, 800, 850, oder 900 nm. Geeignet im Sinne der Erfindung sind aber auch Extrusionsmembranen aus anderen Materialien, z.B. aus Polymeren mit geeigneten Eigenschaften.

Ferner hat sich gezeigt, dass die Qualität der Liposomen, insbesondere die Homogenität der Liposomenmischung, noch weiter verbessert werden konnte, wenn die Extrusion in einem kontinuierlichen Prozess erfolgt und die Lipidsuspension mehrfach durch die Extrusionsmembran, vorzugsweise zwischen 2 - 20 mal, extrudiert wird (vgl. z. B. Ausführungsbeispiel 1, Tabelle 4). Folglich betrifft die vorliegende Erfindung auch Verfahren zur Herstellung von homogenen Liposomen durch Niederdruckextrusion einer Lipidsuspension, vorzugsweise mit einer Lipidkonzentration zwischen 0.04 - 5 mg/ml, bevorzugt zwischen 0.1 - 2 mg/ml, besonders bevorzugt zwischen 0.1 - 1 mg/ml, noch weiter bevorzugt zwischen 0.25 - 1 mg/ml, durch eine 600 - 900 nm Membran, vorzugsweise mit einer Flussrate zwischen 10 - 250 ml/min, besonders bevorzugt zwischen 50 - 150 ml/min, weiter bevorzugt zwischen 75 - 120 ml/min, dadurch gekennzeichnet, dass die Lipidsuspension zumindest 2 mal, vorzugsweise zwischen 2 und 20 mal durch die Membran extrudiert wird. Besonders bevorzugt ist ein entsprechendes Verfahren, bei dem die Extrusion in einem kontinuierlichen Verfahren erfolgt. Noch weiter bevorzugt ist ein entsprechendes Extrusionsverfahren, das in einem geschlossenen System, wie beispielsweise in Figur 1 dargestellt, unter aseptischen Bedingungen durchgeführt wird.

Überraschenderweise hat sich gezeigt, dass das erfindungsgemäße Verfahren besonders geeignet ist zur Herstellung von homogenen kationischen Liposomen /Liposomenmischungen oder von Liposomen / Liposomenmischungen enthaltend ein kationische Lipid und ein neutrales Amphiphil. Insbesondere Liposomen, die aus einer Mischung aus neutralen Amphiphil und kationischem Lipid bestehen, erwiesen sich als besonders homogen und stabil, wenn sie nach dem hier beschriebenen erfindungsgemäßen Verfahren hergestellt wurden.

Unter einem "kationischen Lipid" versteht man ein Lipid das unter gegebenen Bedingungen eine positive Überschussladung besitzt. Unter einem neutralen (zwitterionischen) Amphiphil versteht man ein Molekül das unter gegebenen Bedingungen keine Überschussladung besitzt und somit ladungsneutral ist

Folglich betrifft die vorliegende Erfindung nach einer weiteren Ausführungsform auch Verfahren zur Herstellung von homogenen Liposomen / Liposomenmischungen enthaltend zumindest ein kationisches Lipid oder ein kationisches Lipid und ein neutrales Amphiphil. Geeignete kationische Lipide im Sinne der Erfindung sind beispielsweise DOTMA [N-1-(2,3-dioleyloxy)propyl]-N,N,N,-triemthylammonium chlorid) DORI (1,2-Dioleoyloxycarbonylpropyl-3-dimethylhydroxyethylammonium bromid, DORIE (1,2-Dioleyloxypropyl-3-dimethylhydroxyethylammoniumbromid), DOTAP (Dioleoyltrimethylammoniumpropan(N-[1-(2,3-Dioleoyloxy)propyl]-N,N,N-trimethylammonium-methylsulfat)), DMRIE (N-(1,2-Dimyristoyloxypropyl)-N,N-dimethyl-N-hydroxyethylammonium-bromid)), DOGS (Dioctadecylamidologycyl spermine), DOSPA (2,3-dioleyloxy-N-[2(spermincarboxamido)ethyl]-N,N-diemthyl-1-propanaminiumtrifluoroacetat), PDMAEMA (Poly(2-dimethyl-amino)ethylmethacrylat), DDAB (Dimethyldioctadecylammonium) DC-Chol 3β-[N-(N',N'-Dimethyl aminoethyl)carbamoyl]-cholesterinl) und DAC-Chol ((3-beta[N(N,N'-Dimethylaminoethan)carbamoyl]cholesterin)). Ferner zählen hierzu verschiedene Spermincholesterylcarbamate, wie beispielhaft in WO96/18372, oder 1,4-Dihydropyridine-Derivate, wie beispielsweise in WO01/62946 beschrieben. Eine nicht abschließende Zusammenfassung relevanter kationischer Lipide findet sich beispielsweise in der Publikation von Miller (1998), Angew. Chem. 110, 1862-1880, auf die hier ausdrücklich inhaltlich Bezug genommen wird. Bevorzugt eignet sich das erfindungsgemäße Verfahren zur Herstellung von homogenen Cholesterin-haltigen Liposomen / Liposomenmischungen, vorzugsweise von Liposomen / Liposomenmischungen, die DAC-Chol oder DC-Chol als kationisches Lipid enthalten. Eine Beschreibung von DAC-Chol findet sich u.a auch in WO96/20208, Reszka et al., eine Beschreibung von DC-Chol in US 5,283,185, Epand et al. Einige kationische Lipide und Gemische sind auch kommerziell erhältlich wie Effectene™ und SuperFect™ (Qiagen, Hilden, Deutschland), FuGene 6™ (Roche, Mannheim, Deutschland), LipoFectin™, LipoFectin2000™, LipoFECTAMINE Plus™ (Invitrogen, Karlsruhe, Deutschland).

Geeignete neutrale Amphiphile im Sinne der Erfindung sind beispielsweise Cholinderivate wie Dimyristoylphosphatidylcholin (DMPC), Dipalmitoylphosphatidylcholin (DPPC), Dioleoylphosphatidylcholin (DOPC) **oder** Ethanolaminderivate wie Dimyristoylphosphatidylethanolamin (DMPE), Dipalmitoylphosphatidylethanolamin (DPPE), Dioleoylphosphatidylethanolamin (DOPE), wobei DOPE besonders bevorzugt ist.

Überraschenderweise hat sich gezeigt, dass das hier beschriebene erfindungsgemäße Verfahren zu besonders homogenen Liposomen / Liposomenmischungen mit geringer Polydispersität führt, wenn die zu extrudierende Lipidsuspension DOPE als neutrales Amphiphil und DC-Chol, vorzugsweise DAC-Chol, als kationisches Lipid enthält.

Kationisches Lipid und neutrales Amphiphil können in einem Gewichtsverhältnis von 1:99 bis 99:1 vorliegen, wobei hiermit alle Gewichtsverhältnisse die zwischen diesen Werten liegen umfasst sein sollen. Besonders stabile und homogene Liposomen erhält man, wenn man das kationische Lipid und das neutrale Amphiphil in einem Gewichtsverhältnis von 10:90 bis 40:60, beispielsweise von 10:90, 15:85, 20:80, 25:75, 30:70, 35:65, 40:60 mischt. Weiter bevorzugt sind Mischungsverhältnisse von 21:79, 22:78, 23:77, 24:76, 25:75, 26:74, 27:73, 28:72, 29:71, 30:70, 31:69, 32:68, 33:67, 34:66, 35:65, 36:64, 37:63, 38:62, 39:61, 40:60, insbesondere ein Mischungsverhältnis von 30:70 im Fall von Lipidmischungen aus DC-Chol:DOPE oder DAC-Chol:DOPE. DC-Chol:DOPE bzw. DAC-Chol:DOPE jeweils mit einem Gewichtsverhältnis von 70:30 werden nachfolgend auch im Fall von DC-Chol:DOPE als DC30 und im Fall von DAC-Chol:DOPE als DAC30 bezeichnet. Demnach betrifft die vorliegende Erfindung in einer besonders bevorzugten Ausführungsform die Herstellung von homogenen Liposomen mit einem geringen Polydispersitätsindex von vorzugsweise ≤ 0.6 weiter bevorzugt von ≤ 0.5, und noch weiter bevorzugt von ≤ 0.4, bestehend aus DC-Chol:DOPE, vorzugsweise DAC-Chol:DOPE in einem Gewichtsverhältnis von 30:70 (DC30 bzw. DAC30).

Nach einer weiteren Ausführungsform des erfindungsgemäßen Verfahrens handelt es sich bei der zu extrudierenden Lipidsuspension um eine Suspension eines kationischen Lipids und eines neutralen Amphiphil in einer wässrigen Lösung. Die Lipidsuspension kann darüber hinaus weitere Stoffe, beispielsweise Salze, Polymere, Zucker oder Zuckeralkohole enthalten. Die Beimischung entsprechender Stoffe (= Hilfsstoffe) kann die Stabilität der herzustellenden Liposomen sowie der hieraus hergestellten Liposomen-Nukleinsäure-Komplexe weiter verbessern.

Beispiele für Polymere umfassen Polyvinylpyrrolidone, derivatisierte Zellulosen, wie z.B. Hydroxymethyl-, Hydroxyethyl-, oder Hydroxypropyl-ethylzellulose, polymere Zucker wie z.B. Ficoll oder Dextran, Stärke wie z.B. Hydroxyethyl- oder Hydroxypropylstärke, Dextrine wie z.B. Cyclodextrine (2-Hydroxypropyl-β-cyclodextrin, Sulfobutylether-β-cyclodextrin), Polyethylene, Glykole, Chitosan, Collagen, Hyaluronsäure, Polyacrylate, Polyvinylalkohole und/oder Pektine. Zucker können beispielsweise sein Mono-, Di-, Oligo- oder Polysaccharide oder um eine Kombination hieraus handelt. Beispiele für Einfachzucker sind Fruktose, Maltose, Galaktose, Glucose, D-Mannose, Sorbose und der gleichen. Zweifachzucker sind beispielsweise Laktose, Sucrose, Trehalose, Cellobiose, und der gleichen. Als Mehrfachzucker oder Polysaccharide eignen sich insbesondere Raffinose, Melezitose, Dextrin, Stärke und der gleichen. Als Zuckeralkohole kommen neben Mannitol, Xylitol, Maltitol, Galaktitol, Arabinitol, Adonitol, Laktitol, Sorbitol (Glucitol), Pyranosylsorbitol, Inositol, Myoinositol und der gleichen in Betracht.

Als Salze eignen sich insbesondere pharmazeutisch akzeptable Salze, wie zum Beispiel anorganische Salze wie Chloride, Sulfate, Phosphate, di-Phosphate, Hydrobromide und/oder Nitrat-Salze. Ferner kann die Lipidsuspension auch organische Salze beinhalten, wie z.B. Malate, Maleate, Fumarate, Tartrate, Succinate, Ethylsuccinate, Citrate, Acetate, Lactate, Methansulfonate, Benzoate, Ascorbate, Paratoluensulfonate, Palmoate, Salicylate, Stearate, Estolate, Gluceptate oder Labionat-Salze.

Durch das erfindungsgemäße Herstellverfahren lassen sich homogene Liposomenmischungen bereitstellen, bestehend aus Liposomen mit einer definierten Größe zwischen 250 - 800 nm, vorzugsweise zwischen 280 - 600 nm, besonders bevorzugt zwischen 280 - 500 nm, weiter bevorzugt zwischen 280 - 400 nm, wobei die Liposomen vorzugsweise ein kationisches Lipid und eine neutrales Amphiphil enthalten dadurch gekennzeichnet, dass der Polydispersitätsindex der Liposomenmischung einen Wert von ≤ 0.60, vorzugsweise von ≤ 0.50, weiter bevorzugt von ≤ 0.40. Folglich betrifft die vorliegende Erfindung auch entsprechende Liposomenmischungen bestehend aus Liposomen mit einer definierten Größe zwischen 250 und 800 nm, vorzugsweise zwischen 280 - 600 nm, besonders bevorzugt zwischen 280 - 500 nm, weiter bevorzugt zwischen 280 - 400 nm wobei die Liposomen ein kationisches Lipid und eine neutrales Amphiphil enthalten dadurch gekennzeichnet, dass der Polydispersitätsindex der Liposomenmischung einen Wert von ≤ 0.60, vorzugsweise von ≤ 0.50, weiter bevorzugt von ≤ 0.40 aufweist. Vorzugsweise enthalten die erfindungsgemäßen Liposomen ein Cholesterol-Derivat wie z.B. DC-Chol oder DAC-Chol in Kombination mit einem neutralen Amphiphil ausgewählt aus DMPC, DPPC, DOPC, DMPE, DPPE, oder vorzugsweise in Kombination mit DOPE. Besonders vorteilhaft haben sich entsprechende Liposomen / Liposomenmischungen erwiesen, die DOPE als neutrales Amphiphil und DC-Chol und/oder DAC-Chol als kationisches Lipid enthalten oder aus diesen bestehen, wobei das Massenverhältnis von DOPE zu dem kationischen Lipid 70:30 beträgt (DC30 bzw. DAC30). Darüber hinaus lassen sich durch das erfindungsgemäße Verfahren Liposomen / Liposomenmischungen herstellen, die die oben aufgeführten kationischen Lipide, neutralen Amphiphile, Salze, Polymere, Zucker, Zuckeralkohole oder eine Kombination hieraus enthalten oder aus diesen bestehen.

Die erfindungsgemäßen und hier beschriebenen Liposomen eignen sich zur Herstellung von homogenen Liposomen-Nukleinsäure-Komplexen, zu sog. Lipoplexen, durch einfaches Mischen der entsprechenden Liposomen mit Nukleinsäuremolekülen. Bei den Nukleinsäuremolekülen (= Nukleinsäuren) handelt es sich üblicherweise um genomische DNA, cDNA, synthetische DNA, RNA, mRNA, Ribozyme, antisense-RNA, synthetische Peptidnukleotide und einzelsträngige Oligonukleotide, vorzugsweise um cDNA. Die Nukleinsäure kann zum Beispiel in einen DNA-Expressionsvektor oder in einer Expressionskassette enthalten sein und so die rekombinante Expression eines Gens von Interesse nach Transfektion in einer Zielzelle ermöglichen. Auf diese Art und Weise lassen sich verschiedene Gene, vorzugsweise therapeutische Gene, in eine Zielzelle einschleusen und dort exprimieren. Beispiele für therapeutische Gene sind beispielsweise Insulin, Insulinähnlicher Wachstumsfaktor, humanes Wachstumshormon (hGH) und andere Wachstumsfaktoren, Gewebeplasminogenaktivator (tPA), Erythropoetin (EPO), Cytokine, beispielsweise Interleukine (IL) wie IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, Interferon (IFN)-alpha, beta, gamma, omega oder tau, Tumornekrosefaktor (TNF) wie z.B. TNF-alpha, beta oder gamma, TRAIL, G-CSF, GM-CSF, M-CSF, MCP-1 bis MCP-5, eNOS, iNOS, HO-1, HO-2, HO-3 und VEGF, HGF.

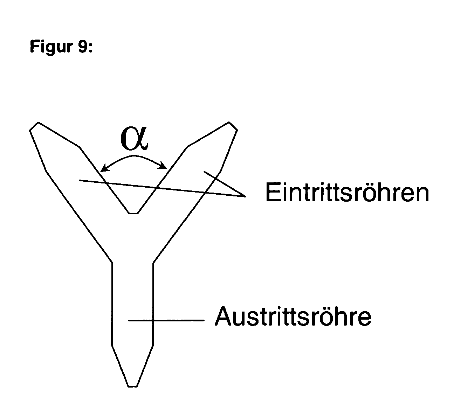

Die Lipoplexherstellung erfolgt üblicherweise durch die Zugabe von Nukleinsäuremolekülen zu Liposomen oder umgekehrt durch die Zugabe von Liposomen zu Nukleinsäuremolekülen. Im Rahmen der vorliegenden Erfindung hat sich insbesondere das gleichmäßige Zusammenführen von Liposomen und Nukleinsäuren als besonders vorteilhaft herausgestellt, beispielsweise über ein sog. Y-Stück. Unter einem Y-Stück versteht man ein 3-schenkliches Rohr, wie beispielhaft in Figur 9 dargestellt. Es besteht aus zwei Eintrittsröhren die über einen spitzen Winkel in eine Ausfuhrröhre zusammengeführt werden. Durch den kontinuierlichen Mischvorgang werden Lipoplexe mit einem konstanten Nukleinsäuregehalt erhalten. Ferner hat sich überraschenderweise gezeigt, dass die kontinuierliche Zusammenführung von Nukleinsäuren und Liposomen zu einer fast vollständigen Internalisierung der Nukleinsäuren in die Liposomen führt, während bei dem einfachen Mischen von Nukleinsäuren und Liposomen, die Nukleinsäuren zumeist aus den Liposomen herausragen und somit weniger gut geschützt sind und als somit die Tendenz haben in Wechselwirkung zu treten und das Ausflocken der Lipoplexe zu induzieren . Folglich ermöglicht die Transfektion von stabilen Lipoplexen, die durch kontinuierliches Mischen über ein sog. Y-Stück erzeugt werden, eine verbesserte Expression in den transfizierten Zielzellen (= verbesserte Bioaktivität). Entsprechend hergestellte Lipoplexe zeichnen sich durch eine hohe Homogenität, physikalische Stabilität in Lösung, eine sehr gute Langzeitstabilität und Lyophilisierbarkeit bei einer hohen Bioaktivität aus. Darüber hinaus ermöglicht das kontinuierliche Zusammenführen von Nukleinsäure und Liposomen die Herstellung von großen Mengen an Lipoplexen, mit gleich bleibend hoher Qualität bis in den Litermaßstab.

Folglich betrifft die vorliegende Erfindung in einer weiteren Ausführungsform ein Verfahren zur Herstellung von Lipoplexen dadurch gekennzeichnet, dass die Mischung von Liposomen und Nukleinsäuremolekülen über ein Y-Stück erfolgt, welches ein gleichmäßiges und kontinuierliches Zusammenführen der Liposomen und Nukleinsäuremoleküle erlaubt. Durch das entsprechende Verfahren, vorzugsweise durch Verwendung eines Y-Stücks, bei dem die beiden Eintrittsröhren, über die die Liposomensuspension und die Nukleinsäuren zusammengeführt werden in einem spitzen Winkel von < 80, vorzugsweise von < 70, weiter bevorzugt von < 60, noch weiter bevorzugt von < 50 bzw. < 40 Grad zueinander stehen (vgl. Figur 9, Winkel α), lassen sich homogene Lipoplexe mit einer Größe von 250 - 600 nm und einem Polydispersität von ≤0.5, vorzugsweise von ≤0.4 herstellen. Der Rohrinnendurchmesser ist jeweils abhängig von den Volumina an Liposomen und Nukleinsäure die durch das Y-Stück zusammen geführt werden. Im Rahmen der vorliegenden Erfindung haben sich Y-Stücke mit einem Rohrinnendurchmesser von 3 bis 5 mm als vorteilhaft herausgestellt. Bei Flussraten von 20 - 800 ml/min, vorzugsweise bei 100 - 500 ml/min ermöglichen entsprechende Y-Stücke ein kontinuierliches Komplexieren von Liposomen und Nukleinsäure zu homogenen Lipoplexen entsprechender Größe. Die in den Ausführungsbeispielen dargestellten Lipoplexe wurden beispielsweise mit einer Flussrate von ungefähr 150 - 170 ml/min bzw. ungefähr 400 ml/min gemischt.

Im Rahmen der vorliegenden Erfindung wurden insbesondere kationische Liposomen, bzw. Liposomenmischungen bestehend aus einem neutralen Amphiphil und einem kationischen Lipid, wie beispielsweise DC30 oder DAC30, zur Herstellung der Lipoplexe verwendet. Die entsprechenden Liposomen tragen somit auf ihrer Oberfläche positive Ladungen, während die Nukleinsäuren aufgrund ihres Phosphatgerüsts negativ geladen sind. Aus WO98/01030 war bekannt, dass ein Ladungsverhältnis von positiv-geladenen Liposomen zur negativ-geladenen Nukleinsäure von 1:20 (+/-), vorzugsweise 2:10 (+/-) einen stabilisierenden Einfluss auf die gebildeten Lipoplexe hat. Vorliegend hat sich gezeigt, dass insbesondere ein Liposomen-Nukleinsäure-Ladungsverhältnis (+/-) von 4 - 0.01, beispielsweise von 4, 3.9, 3.8, 3.7, 3.6, 3.5 usw., 3.0, 2.9, 2.8, 2.7, 2.6, 2.5 usw., 1.0, 1.9, 1.8, 1.7, 1.6, 1.5 usw., 0.9, 0.8, 0.7, 0.6, 0.5 usw, 0.09, 0.08, 0.07, 0.06 usw. vorteilhaft ist und die Stabilität der entstehenden Lipoplexe verbessert. Das Liposomen-Nukleinsäure-Ladungsverhältnis (+/-) gibt hier bei das Verhältnis von positiver Ladung des eingesetzten kationischen Lipid zu der negativen Ladungen der Nukleinsäure wieder. Es wird vorausgesetzt, dass alle einwertig kationischen Lipide eine (1) positive Ladung besitzen. Das bedeutet, dass die Molzahl an eingesetztem kationischen Lipid der Molzahl an positiven Ladungen entspricht (gilt für ein Lipid das nur eine (1) positive Ladung trägt, für mehrwertige kationische Lipide muss dies in der Berechnung berücksichtigt werden). Die Ladungsträger der negativen Ladung auf der Nukleinsäure sind die Phosphatgruppen (eine negative Ladung pro Phosphatgruppe). Als besonders vorteilhaft hat sich ein Liposomen-Nukleinsäure-Ladungsverhältnis (+/-) von 1.25 - 0.75 insbesondere im Zusammenhang mit der Verwendung von DC30-Liposomen oder vorzugsweise mit der Verwendung von DAC30-Liposomen herausgestellt. Folglich betrifft die vorliegende Erfindung auch ein Verfahren zur Herstellung von homogenen Lipoplexen aus kationischen Liposomen, bzw. aus Liposomen die ein kationisches Lipid und ein neutrales Amphiphil enthalten, wie beispielsweise DC30 oder DAC30, über ein Y-Stück, dadurch gekennzeichnet, dass Liposomen-Nukleinsäure-Ladungsverhältnis (+/-) 4 - 0.01, vorzugsweise 2 - 0.1, besonders bevorzugt 1.5 - 0.5 und noch weiter bevorzugt 1.25 - 0.75 beträgt.

Neben dem Mischvorgang selbst, der Flussrate und dem Liposomen-Nukleinsäure-Ladungsverhältnis (+/-) kann die Stabilität der Lipoplexe bei dem Mischvorgang durch die verwendete Liposomenkonzentration positiv beeinflusst werden. Es hat sich herausgestellt, dass beim kontinuierlichen Mischen von kationischen Liposomen, z.B. DC30 oder vorzugsweise DAC30, mit Nukleinsäuren über ein Y-Stück mit einer kontinuierlichen Flussrate von 20 - 800 ml/min, vorzugsweise von 100 - 500 ml/min und einem Liposomen-Nukleinsäure-Ladungsverhältnis (+/-) von 4 - 0.01, vorzugsweise von 2 - 0.1, besonders bevorzugt von 1.5 - 0.5 und noch weiter bevorzugt von 1.25 - 0.75 die Verwendung von einer homogenen Liposomensuspension mit einer Liposomenkonzentration von zwischen 0.02 und 1 mg/ml besonders vorteilhaft ist. Folglich betrifft die vorliegende Erfindung nach einem weiteren Aspekt ein Verfahren zur Herstellung von Lipoplexmischungen durch kontinuierliches Mischen von kationischen Liposomen, z.B. DC30 oder vorzugsweise DAC30, mit Nukleinsäuren über ein Y-Stück mit einer kontinuierlichen Flussrate von 20 - 800 ml/min, vorzugsweise von 100 - 500 ml/min und einem Liposomen-Nukleinsäure-Ladungsverhältnis (+/-) von 4 - 0.01, vorzugsweise von 2 - 0.1, besonders bevorzugt von 1.5 - 0.5 und noch weiter bevorzugt von 1.25 - 0.75, dadurch gekennzeichnet dass eine homogene Liposomensuspension mit einer Liposomenkonzentration von ungefähr 0.02 - 1 mg/ml, vorzugsweise von ungefähr 0.1 - 0.5 mg/ml verwendet wird.

Nach einer weiter bevorzugten Ausführungsform lassen sich die Verfahren zur Herstellung der Liposomen und die Mischung von Liposomen und Nukleinsäuren direkt mit einander koppeln. Dem zu Folge betrifft die vorliegende Erfindung nach einer besonders bevorzugten Ausführungsform ein Verfahren zur Herstellung von homogenen Lipoplexmischungen mit Lipoplexen mit einer Größe von 250 - 600 nm, vorzugsweise 275 - 500 nm, besonders bevorzugt 275 - 400 nm und vorzugsweise einem Polydispersitätsindex von ≤0.5, besonders bevorzugt von ≤0.4, enthaltend die Schritte:

(a) Extrusion einer Lipidsuspension, enthaltend ein kationisches Lipid oder eine Mischung aus einem kationischen Lipid und einem neutralen Amphiphil, beispielsweise DC30 oder vorzugsweise DAC30, in einem kontinuierlichen Verfahren durch eine 600 - 900 nm Membran, vorzugsweise mit einer Flussrate zwischen 10 - 250 ml/min, besonders bevorzugt zwischen 50 - 150 ml/min, weiter bevorzugt zwischen 75 - 120 ml/min, wobei die Lipidkonzentration in der Lipidsuspension vorzugsweise zwischen 0.04 - 5 mg/ml, bevorzugt zwischen 0.1 - 2 mg/ml, besonders bevorzugt zwischen 0.1 - 1 mg/ml, noch weiter bevorzugt zwischen 0.25 - 1 mg/ml beträgt, und die Lipidsuspension zumindest einmal, vorzugsweise aber kontinuierlich zwischen 2 und 20 mal durch die Membran extrudiert wird; **und (b)** die entsprechend hergestellten Liposomenmischung mit Nukleinsäuremolekülen, die zuvor sterilfiltriert wurden, vorzugsweise durch einen 0,2 μm Filter, über ein Y-Stück mit einer kontinuierlichen Flussrate von 20 - 800 ml/min, vorzugsweise von 100 - 500 ml/min und einem Liposomen-Nukleinsäure-Ladungsverhältnis (+/-) von 4 - 0.01, vorzugsweise von 2 - 0.1, besonders bevorzugt von 1.5 - 0.5 und noch weiter bevorzugt von 1.25 - 0.75 gemischt werden.

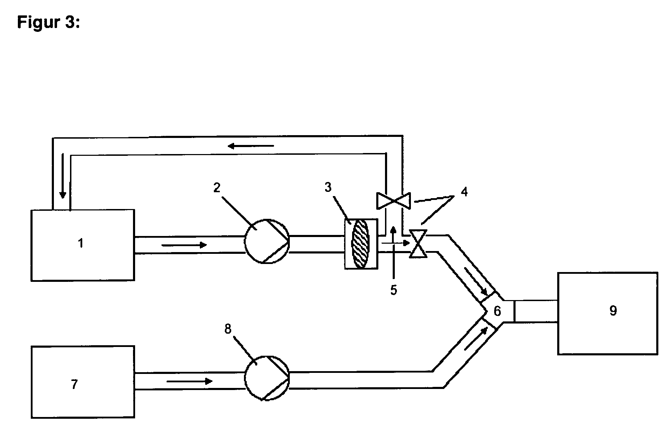

Nach einer weiter bevorzugten Ausführungsform der vorliegenden Erfindung wird dieses "kombinierte" Verfahren in einem geschlossenen System unter aseptischen Bedingungen durchgeführt. Eine Vorrichtung, mit der ein entsprechend kombiniertes Verfahren durchgeführt werden kann ist beispielhaft in Figur 3 dargestellt. Ausgehend von einem Vorlagegefäß (1), das die Lipidsuspension enthält, wird die Lipidsuspension mittels einer Pumpe (2) kontinuierlich durch eine Extrusionsvorrichtung mit einer porösen Membran von 600 - 900 nm (3) gepumpt. Hinter der Extrusionsvorrichtung befinden sich eine Verzweigung (5), die zum einen den Rückfluss der Lipidsuspension in das Vorlagegefäß (1), und somit zum einen eine mehrfache Extrusion der Lipidsuspension ermöglicht oder alternativ die Weiterleitung der extrudierten Lipidsuspension zu dem Y-Stück (6) ermöglicht, über das die Mischung mit der Nukleinsäure erfolgt. Zwischen der Verzweigung (5) und dem Vorlagegefäß (1) sowie zwischen der Verzweigung (5) und dem Y-Stück (6) befindet sich jeweils Ventile (4), über die der Rückfluss oder der Fluss in Richtung Y-Stück geregelt werden kann. In einem zweiten Vorlagegefäß (7) befindet sich die sterilfiltrierte Nukleinsäure, die über eine zweite Pumpe (8) direkt zu dem Y-Stück (6) gepumpt werden kann. Die durch Mischung von extrudierter Lipidsuspension und Nukleinsäure entstandenen Lipoplexe können in einem Auffanggefäß (9) aufgefangen werden. Vorzugsweise handelt es sich bei der Vorrichtung um ein geschlossenes System, so dass der Herstellungsprozess unter aseptischen Bedingungen durchgeführt werden kann. Eine entsprechende Vorrichtung ist ebenfalls Gegenstand der vorliegenden Erfindung.

Durch das hier beschriebene erfindungsgemäße Verfahren war es überraschenderweise möglich den hohen Grad an Homogenität, den die Liposomen aufgrund des speziellen Extrusionsverfahrens aufwiesen, auch nach der Komplexierung mit der Nukleinsäure zu erhalten. Die im Rahmen der vorliegenden Erfindung bereitgestellten Lipoplexmischungen zeichneten sich durch Lipoplexe mit einer Größe von 250 - 600 nm, vorzugsweise von 275 - 500 nm, weiter bevorzugt von 275 - 400 nm und einen geringen Polydispersitätsindex von ≤0.5, vorzugsweise von ≤0.4 und teilweise sogar von ≤0.3 aus. Durch den hohen Grad an Automatisierung konnten chargenübergreifende homogene Lipoplexmischungen erzeugt werden, die sich insbesondere als oder zur Herstellung von Arzneimittel(n) für die gentherapeutische Anwendung eignen. Folglich betrifft die vorliegende Erfindung nach einer weiteren Ausführungsform auch Lipoplexmischungen bestehend aus Lipoplexen mit einer definierten Größe zwischen 250 und 600 nm, wobei die Lipoplexe aus einer Mischung aus homogenen erfindungsgemäßen Liposomen, wie oben beschrieben, und Nukleinsäuremolekülen bestehen dadurch gekennzeichnet, dass der Polydispersitätsindex der Lipoplexmischung einen Wert ≤0.5, vorzugsweise ≤0.4 aufweist. In einer bevorzugten Ausführungsform, handelt es sich bei den Lipoplexen um entsprechende Liposmen-Nukleinsäure-Komplexe, die aus DC30 oder vorzugsweise aus DAC30-Nukleinsäure-Komplexen mit entsprechenden physikalischen Parametern bestehen.

Ein weiterer Aspekt der vorliegenden Erfindung betrifft ein Verfahren zur Lyophilisation der hier beschriebenen erfindungsgemäßen Lipoplexe, vorzugsweise von Lipoplexen enthaltend eine Mischung aus DOPE und DC-Chol oder DOPE und DAC-Chol, vorzugsweise im Verhältnis 70:30 (DC30 bzw. DAC30). Durch das nachfolgend beschriebene Verfahren ist es möglich, die Lipoplexe über längere Zeiten, vorzugsweise über zumindest 8 Monate zu lagern (vgl. Tabelle 20). Wie in den Ausführungsbeispielen dargestellt, unterscheiden sich die Lipoplexe nach ihrer Rekonstituierung weder in ihren physikalischen Parametern noch in ihrer Bioaktivität von nicht lyophilisierten (also "frisch" hergestellten) Lipoplexen. Das erfindungsgemäße Verfahren zur Lyophilisation von Lipoplexen erfolgt in Gegenwart eines geeigneten Stabilisators, vorwiegend in Gegenwart von 250 mM Succrose und 25 mM Natriumchlorid und umfasst folgende Schritte: **(a)** Einfrieren der Lipoplexmischung auf eine Temperatur von ≤ -50°C; **(b)** Trocknen der Lipoplexmischung bei ungefähr - 20°C für zumindest 35 Stunden, vorzugsweise unter Vakuum für 35 - 60 Std. **(c)** Nachtrocknen der Lipoplexmischung bei ungefähr 20°C für zumindest 10 Stunden, vorzugsweise für 10 - 24 Std. Die Zeiten sind hier als Richtzeiten zu verstehen.

Als Stabilisator können beispielsweise verschiedene Zucker, Zuckeralkohole oder Polymere verwendet werden. Diese können als Einzelkommbonente, als Gemisch und/oder in Verbindung mit Salzen verwendet werden. Entsprechende Beispiele für geeignete Zucker, Zuckeralkohole, Polymere oder auch Salze finden sich oben. Vorteilhaft ist die Verwendung des stabilisierenden Mittels in Form einer isoomotischen Lösung (ca. 290 - 330 mOsm), vorzugsweise bei der Herstellung der Liposomen. Die Lipide können beispielsweise vor Extrusion in einer entsprechenden Lösung suspendiert werden, die ein entsprechendes stabilisierendes Mittel enthält. Vorstellbar ist aber auch die Stabilisierung der Lipoplexe durch Zugabe des stabilisierenden Mittels während der Lipoplexherstellung, beispielsweise in dem die Nukleinsäure in einer Lösung aufgenommen wird, die ein entsprechendes stabilisierendes Mittel enthält.

Besonders vorteilhaft für diese Zwecke ist die Verwendung einer Zusammensetzung, die Saccharose als Disaccharid und Natriumchlorid als anorganisches Salz enthält. Ein Beispiel für eine derartige isoosmotische Zusammensetzung (z.B. 300 mOsm) ist eine Kombination von Natriumchlorid in einer Konzentration in einem Bereich von ca. 5 mM bis ca. 100 mM, insbesondere 5, 10, 15, 20, 25, 30, 35, 40, 45 oder 50 mM, mit einem entsprechenden Anteil an Saccharose. Des weiteren ist die Verwendung einer Zusammensetzung enthaltend Mannit allein oder in Kombination mit mindestens einem weiteren Mono- und/oder Disaccharid wie z. B. Saccharose oder Trehalose für die vorgenannten Zwecke bevorzugt. Beispielsweise kann eine derartige isoosmotische Zusammensetzung (z. B. 300 mOsm) eine Kombination von Mannit in einer Konzentration in einem Bereich von ca. 10-290 mM, insbesondere ca. 150-290 mM, und Saccharose bzw. Trehalose entsprechend in einer Konzentration in einem Bereich von ca. 10- 290 mM, insbesondere ca. 10-150 mM, enthalten. Als vorteilhaft hat sich in diesem Zusammenhang die Verwendung von Saccharose in Verbindung mit Natriumchlorid, vorzugsweise 250 mM Saccharose und 25 mM Natriumchlorid, erwiesen.

Als besonders vorteilhaft hat sich ein Verfahren zur Lyophilisation der oben beschriebenen Lipoplexe erwiesen, das die nachfolgenden Schritte umfasst: **(a)** Einfrieren der Lipoplexmischung auf eine Temperatur von ≤ -50°C mit einer Temperaturerniedrigungsrate von ungefähr ≤ 1 °C/min; **(b)** Inkubation der Lipoplexmischung bei ≤ - 50°C für zumindest 2 Stunden; **(c)** Erwärmung der Lipoplexmischung auf ungefähr - 20°C mit einer Erwärmungssrate von ungefähr ≤ 0.3°C/min; **(d)** Trocknen der Lipoplexmischung bei ungefähr - 20°C für zumindest 35 Std., vorzugsweise für 35 - 60 Std. **(e)** Erwärmen der Lipoplexmischung von ca.-20°C auf ca. 20°C mit einer Erwärmungsrate von ungefähr ≤ 0.44°C/min; **(f)** Nachtrocken der Lipoplexmischung bei ca. 20°C für zumindest 10 Std., vorzugsweise 10 - 24 Std. Folglich betrifft die vorliegende Erfindung auch ein entsprechendes Verfahren. Bei der Trocknung (Schritt (d)) haben sich insbesondere Drücke zwischen 0.01 - 0.1 mbar, vorzugsweise zwischen 0.025 - 0.05 mbar als vorteilhaft erwiesen (vgl. Ausführungsbeispiele, Tabellen 11 und 13).

Ferner betrifft die vorliegende Erfindung auch Lipoplexlyophylisate, die nach einem der hier beschriebenen Verfahren hergestellt werden.

Die vorliegende Erfindung umfasst weiterhin die Verwendung der erfindungsgemäßen Lipoplexmischungen, direkt oder in lyophylisierter Form, in der Gentherapie einschließlich einer Kombinationstherapie mit pharmakologischen Wirkstoffen. Dabei kann es zweckmäßig sein, eine Gentherapie mit weiteren therapeutischen Ansätzen zu kombinieren, wie z.B. der Applikation von pharmakologischen Wirkstoffen einschließlich Proteinen und/oder Peptiden.

Üblicherweise werden die Lipoplexe, bzw. eine diese enthaltende pharmazeutische Zusammensetzung mit einer Gesamtdosis in einem Bereich von ca. 0.1 bis ca. 40 µg (einschließlich aller dazwischen liegenden Werte), bezogen auf die Gesamtmenge an Nukleinsäure, verabreicht. In diesem Zusammenhang ist dem Fachmann klar, dass als "dazwischen liegender Wert" jeder Wert zwischen den angegebenen Ober- und Untergrenzen verstanden wird, wie 0.2, 0.3, 0.4, 0.5, 0.6, 0.7, usw.; 1.0, 1.1, 1.2, 1.3, 1.4, 1.5, usw., 2.0, 2.1, 2.2, 2.3, 2.4, 2.5, usw.; 3.0, 3.1, 3.2, 3.3, 3.4, 3.5, usw.; 4.0, 4.1, 4.2, 4.3, 4.4, 4.5, usw.; 5.0, usw.; 6.0, usw.; 7.0, usw.; 8.0, usw.; 9.0, usw.; 10.0, usw.; 11.0, usw., 12.0, usw.; 13.0, usw.; 14.0, usw.; 15.0, usw.; 16.0, usw., 17.0, usw.; 18.0, usw.; 19.0, usw.; 20.0, usw.; 25.0, usw.; 30.0, usw.; 35.0, usw.; 40.0. Es liegt auf der Hand, dass die tatsächlich eingesetzte Dosis, die genaue Zusammensetzung, die Zeit und die Art der Verabreichung sowie die weiteren Einzelheiten der Behandlung variiert werden können. Als ein geeignetes Tiermodell kann entweder das normale Hausschwein (Schwartz, R. S. et al. (1990), Circulation 82, 2190; Karas, S. P. et al. (1992) J. Am. Coll. Cardiol. 20, 467) oder das sog. Minischwein (Tumbleson, M. E. und Schook, L. B. (1996) Advances in swine in biomedical research, Plenum Press, New York, Bd. 2, 684; siehe auch Unterberg, C. et al. (1995) J. Am. Coll. Cardiol. 26, 1747) eingesetzt werden. Die in diesen Modellen ermittelten Ergebnisse können dann auf den Menschen entsprechend übertragen werden. Vorzugsweise wird die pharmazeutische Zusammensetzung mit einer Gesamtdosis in einem Bereich von ca. 0.5 µg bis ca. 10 µg, besonders bevorzugt ca. 1 µg bis ca. 5 µg, jeweils bezogen auf die Gesamtmenge an Nukleinsäure, verabreicht.

**Ausführungsbeispiele**

Die nachfolgenden Beispiele dienen zur weiteren Illustration der erfindungsgemäßen Gegenstände und Verfahren.

Material und Methoden:

Chemikalien und Zellen:

Die verwendeten Hilfsstoffe entsprechen den Anforderungen an pharmazeutisch zugelassene Hilfsstoffe: Saccharose (Südzucker AG, München, DE), Natrium Chlorid (Merck KG, Darmstadt, DE), WFI (water for injection) (Boehringer Ingelheim, Biberach, DE).

Alle verwendeten Lipide sind käuflich erhältlich: DC-Cholesterol and DOPE kann man bei Avanti Polar Lipids, Inc., DAC30 bei G.O.T. Therapeutics Berlin, DE erwerben.

Die verwendeten Plasmide, nachfolgend zum Teil auch einfach als Nukleinsäure bezeichnet, wie z.B. das pMCP-1, ein MCP-1 (monocyte chemoattractent protein 1) kodierendes Plasmid, das pEGFP, ein EGFP (enhancend green fluorescent protein of *A.victoria*) exprimierendes Plasmid, wurden von Boehringer Ingelheim kloniert und hergestellt. Hierzu wurde die kodierende Region von MCP-1 bzw. EGFP hinter einen heterologen Promoter (CMV-Promotor) kloniert. Das Plasmid wies ferner ein Selektionsmarkergen auf (Neomycin-Phosphotransferase-Gen), so dass positiv transfizierte Zellen in Gegenwart ein Selektionsmittels (z.B. G-418) selektioniert werden konnten. Die Plasmide hatten eine Größe von ca. 5 Kilobasenpaare. Die für die Transfektionsversuche verwendeten Zellen BHK21, COS-7, HASMC, A-10 SMC werden von ATCC (American Type Culture Collection) bezogen und nach dem mitgelieferten Protokoll gezüchtet.

Herstellung einer binären Lipidfilms:

Die Herstellung eines binären Lipidfilms erfolgt nach den "Technical Information" Anweisungen von Avanti Polar Lipids Inc. Die beiden Lipide (kationisches Lipid und Helferlipid DOPE) werden in Chloroform oder einem Chloroform:Methanol-Gemisch (2:1 v/v) separat gelöst. Anschließend werden die beiden Lipide in dem gewünschten Massenverhältnis zusammen titriert. Ein Lipidgemisch bestehend aus 30 w% DC-cholesterol und 70 w% DOPE im weiteren als DC30 bezeichnet (die Zahl hinter der Abkürzung des kationischen Lipid gibt dessen Massenverhältnis im Gemisch an). Zum generellen Verfahren vergleiche auch Pleyer et al. Exp. Eye Res. (2001) 73: 1-7). Ein Lipidgemisch bestehend aus 30 w% DAC-cholesterol und 70 w% DOPE wird im weiteren als DAC30 bezeichnet. Das Lipidgemisch wird anschließend steril filtriert. Das binäre Lipidgemisch gelöst im organischen Lösungsmittel wird in eine auf -20°C vorgekühlten Gefriertrocknungsanlage überführt und die Probe equilibriert bis ein Temperaturausgleich der Lösung stattgefunden hat. Die Entfernung des Lösungsmittels erfolgt über Nacht bei -20 °C und einem Druck von 0.94 mbar. Anschließend erfolgt die Entfernung der Restspuren an organischen Lösungsmitteln im Hochvakuum ($10^{-3}$ mbar). Alternativ kann man das organische Lösungsmittel entfernen, indem ein Stickstoff- oder Argonstrom über die Probe (leicht schütteln) geblasen wird und die Probe auf etwa 30 - 40 °C erwärmt wird. Die Entfernung der Restspuren an organischen Lösungsmitteln erfolgt ebenfalls im Hochvakuum. Diese Arbeitsschritte werden unter aseptischen Bedingungen durchgeführt. Aus diesen so hergestellten Lipidfilme können nun Lipidsuspensionen hergestellt werden.

Herstellung einer DC30 Suspension:

Zur Herstellung einer 1 mg/ml Lipidsuspension aus DOPE/DC-Chol 70/30 (w/w) werden 1 ml Transfektionslösung (250 mM Saccharose, 25 mM NaCl) und 1 mg DC30 gemischt und für 30 min bei Raumtemperatur quellen gelassen. Daraus wird eine 0.25 mg/ml DC30 Lipidsuspension durch entsprechende Verdünnung mit Transfektionslösung hergestellt.

Herstellung einer DAC30 Suspension:

Zur Herstellung einer 1 mg/ml Lipidsuspension aus DOPE/DAC-Chol 70/30 (w/w) werden 1 ml Transfektionslösung (250 mM Saccharose, 25 mM NaCl) und 1 mg DAC30 gemischt und für 30 min bei Raumtemperatur quellen gelassen. Daraus wird eine 0.25 mg/ml DAC30 Lipidsuspension wurde durch entsprechende Verdünnung mit Transfektionslösung hergestellt.

Auftauen, Lösen und Sterilfiltration der Nukleinsäure:

Die bei -20°C gelagerte Nukleinsäure (1 mg/ml) wird im Kühlschrank bei 2-8 °C aufgetaut und mit Transfektionslösung (250 mM Saccharose, 25 mM NaCl) auf die gewünschte Konzentration verdünnt. Dabei wird das Plasmid (beispielsweise pMCP-1 oder pEGFP) in die Transfektionslösung eingerührt (0.05 mg/ml). Anschließend erfolgt die Sterilfiltration über ein 0,2 μm Sterilfilter (je nach der zu filtrierende Menge werden verschieden große Sterilfilter verwendet: z.B. Millipak TM 20: 100 cm$^2$ Filterfläche, Millipak TM 40: 200 cm$^2$ Filterfläche, Fa. Millipore). Die Filtration erfolgt über eine Schlauchpumpe. Vor und nach der Sterilfiltration erfolgt ein Integrationstest des Filters (Bubble point Methode, Soll-Bubble-Point: 3.45 mbar, nach Pharm. Eu und nach "GMP-Berater", Nachschlagewerk für Pharmaindustrie und Lieferanten, Oktober 2001, GMP Verlag).

Bestimmung der Teilchengröße und der Polydispersitätsindex

Die Teilchengröße (als mittleren Durchmesser Ø angegeben) als auch der Polydispersitätsindex (PI) der Liposomen und Lipoplexe wird mittels PCS (Photon correlation spectroscopy) bestimmt (Gerät: Malvern Zetasizer 3000 HS, Malvern Autosizer 4700, Malvern Instrument Ltd., Worcestershire, UK, Nicom 380, Nicom Technologies INC, USA).

Alle Geräte wurden mit dem selben Latexstandard kalibriert. Dabei wird die Streuung eines He-Ne-Laser an der Probe in einem Winkel von 90 ° (nach ISO 13321: 1996(E)) gemessen und aus den Streuungsdaten durch Auswertung mit der Kumulantenanalyse die beiden Parameter (Ø und PI) bestimmt. Die Breite der Teilchenverteilung wird durch den dimensionslosen Polydispersitätsindex (PI) beschrieben und ist nach ISO 13321: 1996(E) folgendermaßen definiert:

$$PI = \mu_2 / \langle \Gamma \rangle^2 = \sigma^2 / 2\langle \Gamma \rangle^2$$

Mit $\langle \Gamma \rangle$ mittlere Abklingrate, $\mu_2 = \int (\Gamma - \langle \Gamma \rangle)^2 \, G(\Gamma)d\Gamma$,
$\sigma$ = Standardabweichung (genaueres siehe ISO 13321: 1996(E))

Der mittlere Teilchendurchmesser $X_{PCS}$ (im weiteren nur noch als Ø gekennzeichnet) ist nach ISO 13321: 1996(E) definiert als:

$$1 / x_{PCS} = \int (1 / x) \, G[\, \Gamma(x)] \, d(1/x).$$

Lipidanalytik mittels HPLC (High Performance Liquid Chromatography):

Die Lipidkonzentration als auch das Verhältnis von kationischen Lipid (z.B. DC-Chol) zu Helferlipid (z.B. DOPE) wird mittels HPLC (High Performance Liquid Chromatography) bestimmt. Vgl. hierzu Chang C.D & Harris D. J. (1998) J. Liqu. Chrom. & Rel. Technol. V21: 1119-1136 oder Meyer O., et al. (2000) Eur. J. Pharm. Biopharm. 50: 353-356.

Nukleinsäureanalytik:

Die Qualität der verwendeten Nukleinsäure basierend auf der Beurteilung von ccc-, oc, und linear Anteil wird mittels Agarosegel (Ethidiumbromid-Färbung) nach allgemeinen Angaben bestimmt (vgl. Ausubel, F.M. et al., Current protocols in molecular biology. New York: Green Publishing Associates and Wiley-Interscience. 1994 (*updated*), Sambrook et al., Molecular Cloning: A Laboratory Manual, 2nd Ed., Cold Spring Harbor Laboratory Press, Cold Spring Harbor, N.Y., 1989). Der Gehalt wird mittels PicoGreen Assay oder UV-Spektroskopie bestimmt.

Karl-Fischer Titration:

Die Bestimmung der Restfeucht in Lyophilisatproben erfolgt nach der Methode von Karl Fischer (Europäisches Arzneibuch, 2002).

Bestimmung der Transfektionseffiziens:

Die Proteinexpression der transfizierten Zellen wird mittels eines kommerziell erhältlichen Kits nach der vom Kit-Hersteller vorgeschlagene Anleitung bestimmt: MCP-1: Human MCP-1 ELISA Kit von BD Biosciences Pharmingen, BD Biosciences, San Diego, USA. Die Expression von EGFP wird mittels eines FACS Gerätes (fluorescence activated cell sorter) der Firma Becton Dickinson BD Biosciences, San Jose, USA entsprechend der Herstellerangaben bestimmt (Filter 488 nm).

**Beispiel 1:** Herstellung von homogenen Liposomen DOPE/DC-Chol 70/30 (DC30)

Einfluss der Extrusionszeit/Zyklen auf die Größe und Homogenität der Liposomen:

Zur Herstellung von homogenen Liposomen DOPE/DC-Chol 70/30 wurde eine DC30 Lipidsuspensionen in Transfektionsmedium mit einer Lipid-Konzentration von 1 mg/ml hergestellt (siehe oben). Über ein Vorlagegefäß wurde die Lipidsuspension kontinuierlich über eine Polycarbonat-Membran mit einer Porengröße von 600 nm (Fa. Millipore, Billerica, MA USA) und einer Flussrate von 80 ml/min durch die geschlossene Niederdruckextrusionsapparatur gepumpt (siehe Figur 1). Der gemessene Druck an der Membran betrug weniger als $1 \times 10^5$ Pa ($10^5$ Pa = 1 bar) .

Bei entsprechender Versuchsanordnung entsprach eine Extrusionszeit von ca. 10 min ungefähr 2 Extrusionszyklen.

Geräte:                Schlauchpumpe Filtron
                       Silikonschlauch (Innendurchmesser 4 mm)

Extrusionseinheit:    Filtergehäuse Millipore
                      600 nm Polycarbonatmembran / Ø 47 mm
Vorlagegefäß:         1000 ml Scheidetrichter

Wie man der Tabelle 1 entnehmen kann, wird die Liposomengröße durch die Anzahl der durchgeführten Extrusionen (Extrusionszyklen) gesteuert. Nicht extrudierte Liposomen weisen eine durchschnittliche Größe von über 1500 nm auf, wobei die Streuung, angegeben als ± Standardabweichung (SD) in Bezug auf die durchschnittliche Liposomengröße, ca. ±80% beträgt. Mit zunehmender Anzahl an Extrusionszyklen entstehen Liposomen mit einer durchschnittlichen Größe von ungefähr 600 bis 300 nm, wobei die Homogenität der Liposomen mit zunehmender Anzahl an Extrusionen zunimmt (vgl. Tabelle 1).

Tabelle 1

| Zeit (min) | Größe (nm) | ± SD (nm) | ± SD (%) | Druck (bar) | Zyklen |
|---|---|---|---|---|---|
| 0 | 1606 | 1283 | 80 | --- | 0.0 |
| 0 | 1575 | 1362 | 86 | --- | 0.0 |
| 10 | 607 | 371 | 61 | 0.6 | 2.1 |
| 10 | 606 | 374 | 62 | 1 | 2.1 |
| 15 | 535 | 293 | 55 | 0.6 | 3.2 |
| 15 | 500 | 270 | 54 | 1 | 3.2 |
| 20 | 477 | 269 | 56 | 0.6 | 4.3 |
| 20 | 441 | 247 | 56 | 1 | 4.3 |
| 25 | 435 | 226 | 52 | 0.6 | 5.3 |
| 25 | 407 | 210 | 52 | 1 | 5.3 |
| 30 | 411 | 214 | 52 | 0.6 | 6.4 |
| 30 | 394 | 190 | 48 | 0.9 | 6.4 |
| 35 | 379 | 188 | 50 | 0.6 | 7.5 |
| 40 | 377 | 180 | 48 | 0.6 | 8.5 |
| 45 | 345 | 172 | 50 | 0.9 | 9.6 |

Einfluss unterschiedlicher Flussraten auf die Größe und Homogenität der Liposomen:

Der Prozess wurde analog dem oben beschriebenen Beispiel durchgeführt. Die Flussraten wurden so gewählt, dass der Druck an der Membran einen Wert von 3 x $10^5$ Pa nicht übersteigt. Die Flussraten betrugen 210, 110, 54 ml/min. Das Extrusionsvolumen betrug jeweils 100 ml pro Ansatz. Alle anderen Prozessparameter wurden wie oben beschrieben eingestellt. Wie aus Tabelle 2 ersichtlich, hat die Flussrate einen erheblichen Einfluss auf die resultierende Größenverteilung und Homogenität der Liposomen. Eine Reduktion der Flussrate auf unter 100 ml/min führt bei gleicher Extrusionszeit zu größeren Liposomen.

Tabelle 2:

| Flussrate | 210 ml/min | 110 ml/min | 54 ml/min |
|---|---|---|---|
| Zeit (min) | Größe ± SD | Größe ± SD | Größe ± SD |
| 0 | 1626 nm (1270) | 1804 nm (1360) | 1573 nm (1134) |
| 2 | 423 nm (186) | 557 nm (316) | 689 nm (380) |
| 5 | 371 nm (163) | 429 nm (212) | 522 nm (278) |
| 10 | 358 nm (163) | 374 nm (177) | 436 nm (198) |

Einfluss von Porengrösse, Lipidkonzentration und Anzahl der Extrusionszyklen auf die Größe und Homogenität der Liposomen:

In dem vorgestellten Beispiel wurde die Extrusion mit einer 600 nm- und einer 800 nm- Polycarbonat Membran durchgeführt. Hierzu wurden DC30 Lipidsuspensionen mit einer Lipid-Konzentration von 1 mg/ml bzw. 0.25 mg/ml wie oben beschrieben hergestellt und über ein Vorlagegefäß kontinuierlich über eine Polycarbonat-Extrusionsmembran mit entsprechender Porengröße von 600 nm bzw. 800 nm (Millipore, *supra*) und einer Flussrate von 80 ml/min gepumpt. Der gemessene Druck an der Membran betrug jeweils weniger als $1 \times 10^5$ Pa. Die Beschreibung der Proben ist der Tabelle 3 zu entnehmen. Probe 7 wurde durch Verdünnen der zuvor dreifach extrudierten Probe 6 auf 0.25 mg/ml mit Transfektionslösung hergestellt.

Tabelle 3:

| Probennummer | Probe / Konzentration | Bemerkung |
|:---:|:---|:---|
| 1 | DC30 Lipid, Saccharose-NaCl Lösung Konz. 0.25 mg/mL | 1 Durchlauf |
| 2 | DC30 Lipid, Saccharose-NaCl Lösung Konz. 0.25 mg/mL | 2 Durchläufe |
| 3 | DC30 Lipid, Saccharose-NaCl Lösung Konz. 0.25 mg/mL | 3 Durchläufe |
| 4 | DC30 Liposomen, Konz. 1 mg/mL | 1 Durchlauf |
| 5 | DC30 Liposomen, Konz. 1 mg/mL | 2 Durchläufe |
| 6 | DC30 Liposomen, Konz. 1 mg/mL | 3 Durchläufe |
| 7 | Probe 6 nach 3-facher Extrusion mit Saccharose-NaCl Lösung verdünnt auf 0.25 mg /mL | Verdünnung |

Die vorgestellten Experimente zeigen, dass je nach Wahl der Porengröße der Membran, Extrusionszyklen und Lipidkonzentration die Größe der Liposomen genau eingestellt werden kann. Eine 3-malige Extrusion einer 0.25 mg/ml Lipiddispersion führt je nach Porengröße der Extrusionsmenbran zu verschiedenen Ergebnissen. Bei Verwendung einer 600 nm Membran war die Liposomengrösse 281 nm. Bei Verwendung einer 800 nm Membran war die Liposomengrösse 352 nm. Benutzt man dagegen höhere Lipidkonzentrationen erhält man nach 3 Durchläufen ähnliche Durchmesserwerte nach Verdünnung der Liposomen. Diese Versuche zeigen, dass die drei Prozessparameter Lipidkonzentartion, Extrusionszyklen (-zeit) und Porengrösse der Extrusionsmenbran genau abgestimmt werden müssen. Dabei lassen sich mit zunehmende Anzahl an Extrusionen, Liposomen mit homogener Größenverteilung herstellen (vgl. Tabelle 4). Der Polydisperistätsindex der extrudierten Liposomen verringerte sich mit der Anzahl der Extrusionen. Die Extrusion einer DC30 Lipidsuspension mit 0.25 mg/ml führte zu homogeneren Liposomen im Vergleich zu extrudierten DC30 Lipidsuspensionen mit einer Lipidkonzentration von 1 mg/ml.

Tabelle 4:

| Liposomenprobe | | 600nm-Membran | | 800nm - Membran | |
|:---:|:---|:---:|:---:|:---:|:---:|
| | | Größe (nm) | Poly-Index | Größe (nm) | Poly-Index |
| Probe 1 | 1x extrudiert | 348 | 0.53 | 420 | 0.55 |
| Probe 2 | 2x extrudiert | 290 | 0.40 | 366 | 0.54 |
| Probe 3 | 3x extrudiert | 281 | 0.30 | 352 | 0.45 |
| Probe 4 | 1x extrudiert | 465 | 0.51 | 430 | 0.65 |
| Probe 5 | 2x extrudiert | 390 | 0.56 | 372 | 0.67 |
| Probe 6 | 3x extrudiert | 380 | 0.59 | 355 | 0.57 |
| Probe 7 | Verdünnung | 369 | 0.42 | 368 | 0.46 |

Stabilität der extrudierten Liposomen:

Die durch kontinuierliche Niederdruckextrusion hergestellten Liposomen (Konzentration 1 mg/ml, Flussrate 80 ml/min, Druck kleiner $1 \times 10^5$ Pa, 600 nm Membran) wurden bei Raumtemperatur und bei 4 °C gelagert. Es wurde der Einfluss der Lagerung auf die Stabilität (= Größe) der Liposomen zu unterschiedlichen Zeitpunkten gemessen.

Tabelle 5:

| Extrusionszeit / min | Teilchengröße in nm | | | | | | |
|---|---|---|---|---|---|---|---|
| | Sofort | 6 h | | 12 h | | 24 h | |
| | | RT | 4 °C | RT | 4 °C | RT | 4 °C |
| 5 | 478 | 489 | 482 | 463 | 471 | 487 | 482 |
| 10 | 397 | 389 | 392 | 386 | 395 | 379 | 385 |
| 15 | 368 | 372 | 379 | 375 | 368 | 381 | 375 |
| 30 | 352 | 348 | 346 | 356 | 351 | 360 | 354 |

Man erkennt, dass die Größe der Liposomen über 24 Stunden bei 4°C und bei Raumtemperatur stabil bleibt. Es werden also keine "Frustrationen" (Oberflächenspannungen) in die Liposomen eingebaut, die zu Fusion führen würden. Aus den durchgeführten Experimenten und vorgestellten Beispiel ergibt sich: Die kontinuierliche Durchlaufextrusion bei niedrigen Druck (kleiner $3 \cdot 10^5$ Pa = 3 bar) ist ein geeignetes Verfahren zur reproduzierbaren Herstellung von Liposomen eines Größenbereiches zwischen ca. 250 und 800 nm. Die Größe der Liposomen kann durch die Extrusionsdauer, die Flussrate, und die Porengröße der Extrusionsmembran eingestellt werden. Die Liposomengröße bleibt über einen Zeitraum von mind. 24 Stunden sowohl bei Raumtemperatur als auch bei 4 °C stabil und erlaubt so eine sichere und einfache "Weiterverarbeitung" der Liposomen zu Lipoplexen. Der Prozess kann großtechnisch mit einfachen Mitteln im Sinne eines stufenlos "scalebaren" und aseptischen, validierbaren Verfahrens umgesetzt werden. Während des Extrusionsverfahrens (600 nm Extrusionsmembran) bleibt die Qualität der DC30 Liposomen unverändert. Weder die Konzentration noch das Verhältnis DC-Chol zu DOPE wird verändert. Der Lipidgehalt der Liposomen (Tabelle 6) wurde mittels HPLC (high performance liquid chromatography) bestimmt.

Tabelle 6:

| Probe | Zusammensetzung (Soll) | Charge 1 1 Extrusion (μg/mL) | Charge 2 2 Extrusionen (μg/mL) | Charge 3 3 Extrusionen (μg/mL) |
|---|---|---|---|---|
| Liposomen nach Extrusion | DC-Chol 30 μg/mL | 33.4 | 30.8 | 31.4 |
| | DOPE 70 μg/mL | 72.1 | 68.3 | 69.7 |

**Beispiel 2:** Herstellung von homogenen Liposomen DOPE/DAC-Chol 70/30 (DAC30)

Auf Grundlage der Erkenntnisse aus Beispiel 1 wurden homogene Liposomen mit einer Größe zwischen 250 und 800 nm und einem Polydispersitätsindex von ≤ 0.6 hergestellt.

Zur Herstellung von homogenen Liposomen DOPE/DAC-Chol 70/30 w/w (DAC30) wurde ein DAC30 Lipidfilm 30 min mit Transfektionsmedium (250 mM Saccharose, 25 mM NaCl) inkubiert und 30 min quellen gelassen. Die Lipid-Konzentration wurde auf 1 mg/mL eingestellt. Die Lipidsuspension wurde in die Niederdruckextrusionsapparatur überführt. Anschließend wurde die Lipidsuspension kontinuierlich und gleichmässig (analog zu Beispiel 1) über eine Polycarbonatmembran mit einer Porengrösse von 800 nm (Fa. Millipore, Billerica, MA USA) und einer Flussrate von 80 ml/min durch die geschlossene Niederdruckextrusionsapparatur gepumpt (siehe Figur 1). Der gemessene Druck an der Membran betrug weniger als $1 \times 10^5$ Pa. Bei entsprechender Versuchsanordnung entsprach eine Extrusionszeit von 5 min einem (1) Extrusionszyklus. Das Experiment wurde unter aseptischen Bedingungen mit sterilen Materialien und Apparaturen durchgeführt.

Geräte: Schlauchpumpe Filtron
Silikonschlauch (Innendurchmesser 4 mm)
Extrusionseinheit: Extrusionsgehäuse Gelman Sciences 2220
800 nm Polycarbonatmembran / Ø 47 mm
Vorlagegefäß: 1000 ml Scheidetrichter

Danach wurden die extrudierten DAC30 Liposomen mit sterilfiltriertem Transfektionsmedium (250 mM Saccharose, 25 mM NaCl) auf eine Lipid-Konzentration von 0.25 mg/ml verdünnt.

Nach dem Quellen des Lipidfilms erhält man Liposomen einer Größe von 500 bis 1500 nm, deren Größe von Charge zu Charge meistens nicht reproduziert werden kann (vergleiche Tabelle 7). Nach einer einmaligen Extrusion der 1 mg/mL Lipidsuspension durch die 800 nm Polycarbonatmembran erhielt man DAC30 Liposomen einer Größe von 430 bis 450 nm mit einem schmalen Polydispersitätsindex (= PI) im Bereich von 0,.4 bis 0.5. Die Größe der Liposomen nach Verdünnung der 1 mg/ml extrudierten Liposomen auf eine Endkonzentration von 0.25 mg/ml lag im Bereich von 420 - 440 nm (PI = 0.4 bis 0.5)..

Tabelle 7:

| Experiment | Vor Extrusion | Nach Extrusion 1 mg/ml | Nach Verdünnung 0.25 mg/ml |
|---|---|---|---|
| | Größe (PI) | Größe (PI) | Größe (PI) |
| 1 | 1100nm (0.99) | 447 nm (0.47) | 439 nm (0.40) |
| 2 | 502 nm (0.96) | 434 nm (0.45) | 423 nm (0.49) |

Die so hergestellten Liposomen können über mehrere Tage (7 Tage) bei 2-8 °C gelagert werden ohne ihre Qualität einzubüßen. Nach 7 Tage Lagerung bei 2-8 °C betrug die Liposomengrösse (Experiment 2 aus Tabelle 7, 0.25 mg/mL) 425 nm (PI = 0.48).

**Beispiel 3:** Herstellung von homogenen Lipoplexen bestehend aus DOPE/DC-Chol 70/30 (DC30) und Nukleinsäure

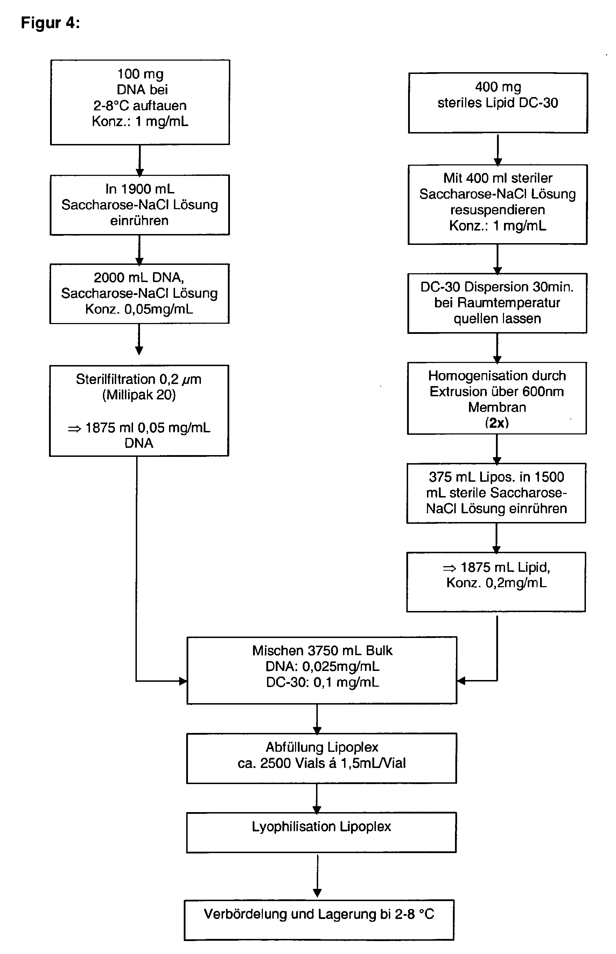

Auf Grundlage der Erkenntnisse aus Beispiel 1 wurden homogene Liposomen hergestellt. Im Fliessschema (Figur 4) sind die einzelnen Schritte für die Herstellung der DC30 Lipoplexe dargestellt. In dem vorgestellten Beispiel betrug das Massenverhältnis Lipid zu DNA 4:1 was einem Ladungsverhältnis +/- von etwa 0.75 entspricht.

400 mg steriles Lipid DC30 wurden mit 400 ml steriler Transfektionslösung (250 mM Saccharose und 25 mM NaCl) versetzt, so dass die Lipidkonzentration 1 mg/ml betrug. Nach 30 min. quellen bei Raumtemperatur erfolgte die zweimalige Extrusion über eine 600 nm Polycarbonatmembran. 375 ml dieser Lipidsuspension wurden mit 1500 ml Transfektionslösung versetzt. Dabei erhielt man 1875 ml einer 0,2 mg/mL DC30 Lipidsuspension. Die Liposomen hatten eine Größe von 310 nm (PI = 0.35).

Bei 2-8 °C aufgetaute DNA (100 mg) einer Konzentration von 1 mg/ml wurde in 1900 ml Transfektionslösung eingerührt. Die DNA-Konzentration betrug nach dem Verdünnungsschritt 0.05 mg/mL. Die DNA-Lösung wurde anschließend steril filtriert. 1875 ml dieser steril filtrierten DNA-Lösung wurde im nächsten Schritt mit den DC30 Liposomen gemischt. Die Ausgangsvolumina der Liposomensuspension und DNA-Lösung betrugen jeweils 1875 ml und die Konzentrationen der Liposomensuspension (0.2 mg/mL) und DNA-Lösung (0.05 mg/mL) war so eingestellt, dass das Massenverhältnis Lipid zu DNA 4:1 beträgt.

Stabile Lipoplexe erhielt man bei Anwendung des in den Figuren 3 und 4 dargestellten Verfahrens. Dabei muss der vorgestellte Mischungsprozess so gefahren werden, dass ein gleichmäßiges und kontinuierliches Zusammenführen von Liposomen und Nukleinsäuren gewährleistet wird. Hierzu wurde die jeweilige Liposomensuspension und DNA-Lösung über ein Y-Stück (z.B. Hibiki Y-1 Ø 3 mm, Hibiki Y-2 Ø 5 mm für größere Volumina, oder Y-Stück Norma Ø 3 mm und Ø 5 mm) gleichmäßig und kontinuierlich gemischt und in eine sterile Flasche überführt. Andere Geometrien sind auch möglich. Das verwendete Y-Stück hatte einen Durchmesser von 3 mm (Figur 3). Die Y-Stücke können aus Polypropylen (PP), Polyethylene (PE) oder Polyvinylchlorid (PVC) oder aber aus Edelstahl oder Glas sein. Die beiden Pumpen für die Nukleinsäurelösung und für die Liposomensuspension müssen "gleich geschaltet" werden (= gleiche Flussraten), damit ein gleichmäßiges und kontinuierliches Pumpen der Lösungen durchführbar ist. Bei Verwendung einer einzigen Pumpe die es erlaubt 2 Flüssigkeiten separat zu befördern, muss man darauf achten, dass beide Flüssigkeiten gleichzeitig angesaugt werden und über das Y-Stück zusammengeführt und gemischt werden. Die Durchflussrate betrug in dem vorgestellten Beispiel 150 - 170 ml/min. Das Volumen der beiden Ausgangslösungen war gleich.

Geräte: Ismatec Schlauchpumpe

Y-Stück: Ø 3mm, Hibiki Y-1 (Carl Roth GmbH & Co.KG., Karlsruhe, DE)

2 Silikonschläuche Fa. ITE (Ø 4 mm) (Intertechnik Elze, Elze, DE)

Nach dem Mischen wurde der Lipoplex Bulk (3750 ml) mindestens 30 min bei Raumtemperatur stehengelassen, bevor die Weiterverarbeitung erfolgte: Konzentration DNA: 0.025 mg/mL; Konzentration Lipid: 0.1 mg/ml.

Nach 30 min nach der Herstellung der Lipoplexe betrug die Lipopolexgröße in der Regel 270 - 310 nm und veränderte sich innerhalb von 3 Std. Lagerung bei Raumtemperatur kaum (Tabelle 8).

Tabelle 8:

| Experiment | 1 | | 2 | |
|---|---|---|---|---|
| | Größe (nm) | Poly-Index | Größe (nm) | Poly-Index |
| Nach 0.5 Std. | 297 | 0.15 | 289 | 0.17 |
| Nach 3 Std. | 275 | 0.15 | 278 | 0.15 |

In der nächsten Tabelle (Tabelle 9) sind PCS Ergebnisse (nach 30 min gemessen) von Liposomen (DC30) und Lipoplexen (DC30/DNA 4:1 w/w) dargestellt, die nach dem oben beschriebenen Mischverfahren hergestellt wurden. Die Liposomensuspension wurde durch verschiedene Extrusionszyklen (600 nm Extrusionsmembran) erhalten. Man erkennt, dass die Ausgangsbedingungen einen Einfluss auf die Qualität der Lipoplexe haben. Aus den Daten der Tabellen 8 und 9 ist ersichtlich, dass eine zweimalige Extrusion der Liposomen durch dieselbe Extrusionsmembran Liposomen liefert, mit denen man Lipoplexe herstellen kann, die im Größenbereich von 280 - 310 nm mit PI < 0.3 liegen.

Tabelle 9:

| | Probe Charge 1 1 Extrusion | | Charge 2 2 Extrusionen | | Charge 3 1 Extrusion | |
|---|---|---|---|---|---|---|
| | Grösse (nm) | Poly-Index | Grösse (nm) | Poly-Index | Grösse (nm) | Poly-Index |
| Liposomen nach Extrusion | 414 | 0.43 | 338 | 0.35 | 323 | 0.31 |
| Lipoplex vor Lyophilisation | 338 | 0.30 | 281 | 0.20 | 269 | 0.16 |
| Lipoplex nach Lyophilisation | 338 | 0.31 | 284 | 0.22 | 259 | 0.18 |

**Beispiel 4:** Herstellung von homogenen Lipoplexen bestehend aus DOPE/DAC-Chol 70/30 (DAC30) und Nukelinsäure

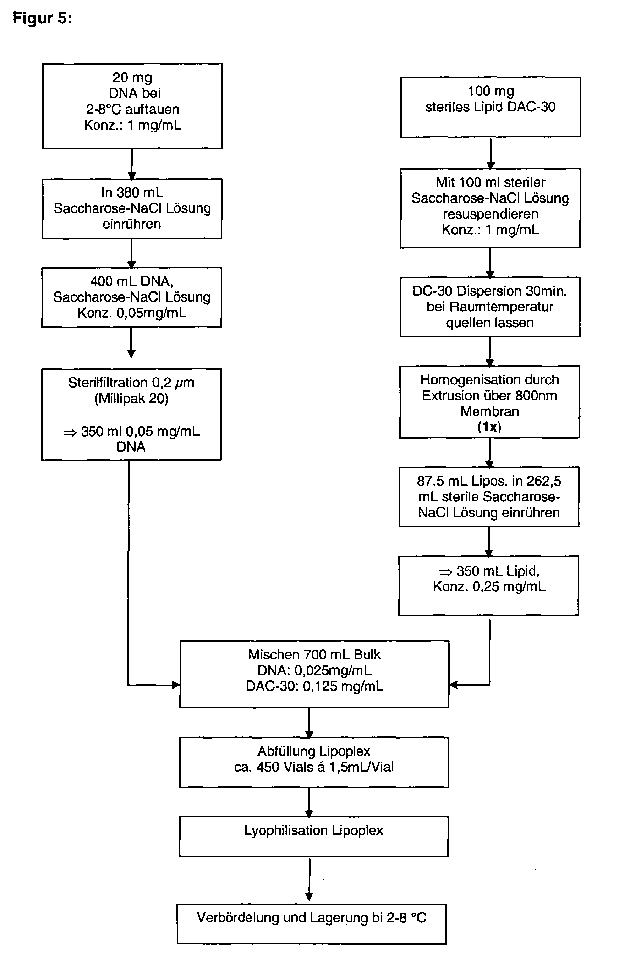

Auf Grundlage der Erkenntnisse aus Beispiel 2 wurden homogene Liposomen hergestellt. Im Fließschema (Figur 5) sind die einzelnen Schritte für die Herstellung der DAC30 Lipoplexe dargestellt. In dem vorgestellten Beispiel betrug das Massenverhältnis Lipid zu DNA 5:1 was einem positiv zu negativ Ladungsverhältnis +/- von etwa 1 entspricht. Hierzu wurden 100 mg steriles Lipid DAC30 mit 100 ml steriler Transfektionslösung (250 mM Saccharose und 25 mM NaCl) versetzt, so dass die Lipidkonzentration 1 mg/ml betrug. Nach 30 min. quellen bei Raumtemperatur erfolgte die einmalige Extrusion über eine 800 nm Polycarbonatmembran. 87.5 ml dieser Lipidsuspension wurden mit 262.5 ml Transfektionslösung versetzt, so dass man dabei 350 ml einer 0.25 mg/ml DC30 Lipidsuspension erhielt. Die Liposomen hatten eine Größe von etwa 430 nm (PI = 0.4).

Bei 2-8 °C aufgetaute DNA (20 mg) einer Konzentration von 1 mg/mL wurden in 380 ml Transfektionslösung eingerührt. Die DNA-Konzentration betrug nach dem Verdünnungsschritt 0.05 mg/ml. Die DNA-Lösung wurde anschließend steril filtriert. Diese steril filtrierten DNA-Lösung wurde im nächsten Schritt mit den DAC30 Liposomen gemischt. Die Ausgangsvolumina der Liposomensuspension und DNA-Lösung betrug jeweils 350 ml. Die Konzentrationen der Liposomensuspension (0.25 mg/ml) und DNA-Lösung (0.05 mg/ml) waren so eingestellt, dass das Massenverhältnis Lipid zu DNA 5:1 beträgt.

Stabile Lipoplexe erhält man bei Anwendung des in den Figuren 3 und 5 dargestellten Verfahrens. Dabei muss der vorgestellte Mischungsprozess so gefahren werden, dass ein gleichmäßiges und kontinuierliches Zusammenführen von Liposomen und Nukleinsäuren gewährleistet wird siehe Beispiel 3). Die jeweilige Liposomensuspension und DNA-Lösung wurden über ein Y-Stück (Figur 9) gleichmäßig und kontinuierlich gemischt und in eine sterile Flasche überführt. Das verwendete Y-Stück hatte einen Durchmesser von 3 mm. Die beiden Pumpen für die Nukleinsäurelösung und für die Liposomensuspension wurden "gleich geschaltet" damit ein gleichmäßiges und kontinuierliches Pumpen der Lösungen durchführbar ist. Bei Verwendung einer einzigen Pumpe, die es erlaubt 2 Flüssigkeiten separat zu befördern. Man muss darauf achten, dass beide Flüssigkeiten gleichzeitig angesaugt werden und über das Y-Stück zusammengeführt und gemischt werden. Die Durchflussrate betrug in dem vorgestellten Beispiel 400 ml/min. Das Volumen der beiden Ausgangslösungen ist gleich.

Geräte: Ismatec Schlauchpumpe
Y-Stück: (Ø 3mm, Hibiki Y-1 (*supra*)
2 Silikonschläuche Fa. ITE (Ø 4 mm)

Nach dem Mischen wurde der Lipoplex Bulk (700 ml) mindestens 30 min bei Raumtemperatur stehengelassen, bevor die Weiterverarbeitung erfolgt: Konzentration DNA: 0.025 mg/mL; Konzentration Lipid: 0.125 mg/mL.
Die Qualität der nach Beispiel 4 hergestellten Lipoplexe ist in Tabelle 10 zusammengefasst. Dargestellt sind die PCS Ergebnisse von 4 verschiedenen Chargen die nach dem beschriebenen Verfahren hergestellt wurden.

Tabelle 10:

| Charge | Größe (nm) | Poly-Index |
|--------|-----------|-----------|
| 1 | 309 | 0.27 |
| 2 | 300 | 0.28 |
| 3 | 312 | 0.24 |
| 4 | 320 | 0.23 |

Diese Beispiele zeigen eindeutig, dass mit dem vorgestellten Verfahren reproduzierbare Chargen hergestellt werden können. Die Lipoplexe liegen im Bereich von 280 - 330 nm mit PI < 0.4.

**Beispiel 5:** Abfüllung von homogenen Lipoplexen bestehend aus DOPE/DC-Chol 70/30 (DC30) und Nukleinsäure

Die Abfüllung der im Beispiel 3 hergestellten DC30 Lipoplexe erfolgte über eine Standard Abfüllmaschine (Fa. Bausch & Ströble, GmbH & Co. KG., Ilshofen, DE) nach dem Abfüllprinzip der Kolbenpumpe in ein 2 ml Vial (Füllmenge 1,5 ml, vergleiche Figur 4). Auch dieser Arbeitsschritt erfolgte mit sterilen Materialien unter aseptischen Bedingungen. Als Primärpackmittel wurde benutzt:

Vial: Injektionsflasche 2R farblos GA1 BB (Fa. Schott, Mainz, DE)
Stopfen: Gusto 13 mm V2 F210 3WRS D713
Bördelkappe: Kombika/Alu 13mm (jeweils Fa. The West Company, Deutschland GmbH, Eschweiler, DE)

**Beispiel 6:** Abfüllung von homogenen Lipoplexen bestehend aus DOPE/DAC-Chol 70/30 (DAC30) und Nukleinsäure

Die Abfüllung der im Beispiel 4 hergestellten DAC30 Lipoplexe erfolgt über eine Standard Abfüllmaschine (Fa. Bausch & Ströble, *supra*) nach dem Abfüllprinzip der Kolbenpumpe. Alternativ kann man bei geringen Bulkvolumina eine händische Abfüllung mittels Eppendorf-Pipetten durchführen. Auch dieser Arbeitsschritt erfolgt mit sterilen Materialien unter aseptischen Bedingungen (Packmittel wie in Beispiel 5).

**Beispiel 7:** Lyophilisation von homogenen Lipoplexen bestehend aus DOPE/DC-Chol 70/30 (DC30) und Nukleinsäure

Vorversuche zur Lyophilisation:

Der Prozess der Lyophilisationsführung hat einen erheblichen Einfluss auf die Qualität der Lyophilisate. Die vorgestellten Lyophilisationsversuche von Lipoplexe wurden in der Gefriertrocknungsanlage (Lyophilisator) Lyo Com 4018 (Fa. Hof) durchgeführt. Die technischen Details der Gefriertrocknungsanlage sind in Tabelle 11 zusammengefasst. Die Ausgangsdaten für die Prozessführung der Lyophilisation (Start-Werte) sind in Tabelle 12 dargestellt. Der Gesamtzeit des Startprozesses dauert 53.5 Stunden.

Tabelle 11:

| Anlagen Bezeichnung | GFT-Anlage |
|---------------------|------------|
| Model und Hersteller | Lyo Com 4018 (Tiny Lyo) Hof GmbH 35102 Lohra |
| Gesamte Stellfläche | 0,36 m$^2$ |
| Anzahl Stellflächen | 3 |
| Kondensator Position | extern, unterhalb der Trocknungskammer |

Tabelle 11:   (fortgesetzt)

| | |
|---|---|
| Kondensator max. Eiskapazität | 10-15 kg |
| Niedrigste Stellflächentemperatur | -55°C |
| Niedrigste Kondensatortemperatur | -80°C |
| Vakuumregelung | Pirani Vakuumventil |
| Temperaturfühler | PT 100 |

Tabelle 12:

| | Dauer (hh: mm) | Sol-Temperatur (°C) | Vakuum (mbar) |
|---|---|---|---|
| **Beladen** | 00:05 | 5 | 1000 |
| **Einfrieren** | 00:55 | -50 | 1000 |
| | 00:30 | -50 | 1000 |
| | 01:00 | -50 | 1000 |
| | 00:30 | -50 | 1000 |
| **Haupttrocknung** | 01:30 | -20 | 0,05 |
| | 33:30 | -20 | 0,05 |
| | 3:30 | -20 | 0,05 |
| **Nachtrocknung** | 01:30 | 20 | 0,05 |
| | 10:00 | 20 | 0,05 |
| **Entnehmen** | 00:30 | 5 | 0,05 |
| **Gesamtzeit** | **53:30** | ------- | |

Der Lyophilisator wurde am Ende des Lyophilisationsprozess mit Stickstoff belüftet und die Vials wurden bei 600 mbar geschlossen. Ausgehend von den Prozessparameter die in Tabelle 12 zusammen gefasst sind, wurden für die Versuchsdurchführung der Versuche 1 - 5 (V01 - V05) die in Tabelle 13 zusammengetragen Änderungen vorgenommen, um den Prozess zu optimieren.

Tabelle 13:

| Versuch | Einfrieren | Haupttrocknung (HT) | Nachtrocknung (NT) | Änderungen Programm |
|---|---|---|---|---|
| V01 | in 30 min von 5 auf -50 °C 4 h halten | **0.10 mbar** in 30 min auf **-30** °C 23.5 h halten in 1 h auf 0 °C 13.5 h halten | **0.01 mbar** in 1.5 h auf 20 °C 4 h halten | Kühlrampe auf -50 °C |
| V02 | in 30 min von 5 auf -50 °C 4 h halten | **0.05 mbar** in 1 h auf **-10** °C 23 h halten in 1 h auf 0 °C 13.5 h halten | **0.01 mbar** in 1.5 h auf 20 °C 4 h halten | HT tieferes Vakuum, Erhöhung der Temperatur |
| V03 | in 55 min von 5 auf -50 °C 4 h halten | **0.025 mbar** (Ist 0.05-0,025 mbar) in 2 h auf **-10** °C 36.5 h halten | **0.01 mbar** in 1.5 h auf 20 °C 4 h halten | Einfrierrampe 1 °C/min HT verlängert 0,025 mbar Vakuum |
| V04 | in 55 min von 5 auf -50 °C 4 h halten | **0.05 mbar** in 1.5 h auf **-20** °C 37 h halten | **0.05 mbar** in 1.5 h auf 20 °C 4 h halten | HT bei -20°C HT und NT identisches Vakuum |

Tabelle 13: (fortgesetzt)

| Versuch | Einfrieren | Haupttrocknung (HT) | Nachtrocknung (NT) | Änderungen Programm |
|---------|-----------|--------------------|--------------------|---------------------|
| V05 | in 55 min von 5 auf -50 °C **2 h** halten | **0.05 mbar** in 1.5 h auf **-20 °C** 37 h halten | **0.05 mbar** in 1.5 h auf 20 °C 8+2 h halten | Einfrieren 2h (Haltephase verkürzt) Variation NT-Zeit |

Die optische Beurteilung der Lyophilisationskuchen der einzelnen Lyophilisate ist in Tabelle 14 zusammengefasst. Aus der Beurteilung der Optik der Lyophilisationskuchen ergab sich, dass der Lyophilisationskuchen des Versuchs V01 ungeeignet war, da eine erhebliche Anzahl an Lyophilisationskuchen kollabiert war. Solche Lyophilisationskuchen hatten sehr lange Rekonstitutionszeiten. Die Restfeuchten, die mittels Karl-Fischer Titration bestimmt wurden, sind in Tabelle 16 zusammen getragen. Die Restfeuchten der Lyophilisationskuchen von Versuch V01 lagen bei über 5%.

Tabelle 15:

| Versuch | Kuchencharakteristik | Beurteilung |
|---------|---------------------|-------------|
| V01 | Lyokuchen in der Mitte der Teilbeladung sind vollständig kollabiert, Lyokuchen im Randbereich sind stark geschrumpft. | ungeeignet |
| V02 | Alle Lyokuchen sind stark geschrumpft, wenige Kuchen mit Hohlräumen =kollabiert Kein deutlicher Unterschied zwischen außen und innen im Blech. | geeignet (außer kollabierte Kuchen) Optisch grenzwertig |
| V03 | wie V02 , ~10% Kuchen (verstreut über Beladung) kollabiert. | geeignet (außer kollabierte Kuchen) Optisch grenzwertig |
| V04 | Kuchen geschrumpft, kein Unterschied innen / außen | Geeignet Optisch grenzwertig |
| V05 | Kuchen geschrumpft, kein Unterschied innen / außen | Geeignet Optisch grenzwertig |

Lyophilisate die nicht kollabiert waren (aus V01) zeigen eine geringere Restfeuchte. Die Versuche V03 und V04 wurden durchgeführt mit verschiedenen Formulierungen. Die eine Formulierung enthielt Natrium Chlorid (250 mM Saccharose, 25 mM NaCl), die andere enthielt kein Natrium Chlorid (250 mM Saccharose). Aus den Daten der Tabelle 16 erkennt man, dass die Abwesenheit von Natrium Chlorid zu trockeneren Lyophilisate führt. Die Abwesenheit von Natrium Chlorid in der Formulierung führt jedoch zu Instabilitäten der Lipoplex-Flüssigformulierung, so dass Natrium Chlorid notwendig war. Durch längere Zeiten für die Nachtrocknung gelingt die Herstellung von Lyophilisaten mit Restfeuchten deutlich unterhalb von 3% (V05 aus Tabelle 16).

Tabelle 16:

| Versuch | NT | Probe | n | Mittelwert % | Min % | Max % | Std | Vk % |
|---------|-----|-------|---|--------------|-------|-------|-----|------|
| V01 | 20°C, 4 h | schlecht | 3 | 559 | 529 | 5.85 | 0.28 | 5 |
| V02 | 20°C, 4 h | gut (innen) | 3 | 320 | 2.61 | 3.57 | 0.52 | 16 |
| V03 | 20°C, 4 h | gut | 3 | 274 | 222 | 3.37 | 0.58 | 21 |
| | 20°C, 4 h | gut/ohne NaCl | 3 | 158 | 1.26 | 1.90 | 0.32 | 20 |
| V04 | 20°C, 4 h | gut | 5 | 290 | 2.39 | 3.27 | 0.35 | 12 |
| | 20°C, 4 h | gut/ohne NaCl | 5 | 132 | 1.13 | 1.47 | 0.13 | 10 |
| V05 | 20°C, 8 h | gut | 5 | 223 | 2.02 | 2.74 | 0.29 | 13 |
| | 20°C, 10h | gut | 5 | 196 | 1.50 | 2.70 | 0.45 | 23 |

Die Daten für die Produkttemperatur der einzelnen Versuche zeigt Tabelle 17.

Tabelle 17:

| Versuch | HT | Anfang HT [°C] | bei Trocknungssprung (°C) | Mittlere Temperatur (°C) |
|---|---|---|---|---|
| V01 | -30 °C/0.10 mbar | -42 | -30 | -36 |
| V02 | -10 °C/0.05 mbar | -40 | -18 | -29 |
| V03 | -10 °C/0.025 mbar | -40 | -27 | -34 |
| V04 | -20 °C/0.05 mbar | -40 | -26 | -33 |
| V05 | -20 °C/0.05 mbar | -42 | -30 | -36 |

Die Temperatur des Glasübergangs (Tg) der beiden Formulierungen wurde kalorimetrisch bestimmt (DSC 821 Fa. Mettler Toledo (Giessen, DE)):

| Tg' Wendepunkt 10 °C/ min | Placebo | -34 °C |
|---|---|---|
| | Placebo ohne NaCl | -32 °C |
| | Verum | -33 bis -35 °C |

Die Haupttrockung soll bei einer Temperatur die unterhalb des Glasübergangs liegt, durchgeführt werden. Zusammenfassend lässt sich sagen, dass optisch kollabierte Kuchen eine höhere Restfeuchte aufwiesen als nicht kollabierte Kuchen. Lyophilisate ohne NaCl war stets trockener als Lyophilisate mit NaCl. Mit zunehmender Nachtrocknungszeit nahm die Restfeuchte ab, nach 10 Std. bei 20 °C lag die Restfeuchte bei ca. 2 %. Verum und Placebo waren vergleichbar in der Restfeuchte. Am Anfang der Haupttrocknung lag die Produkttemperatur (-40°C) unterhalb des Tg (-34°C). Bei den Haupttrocknungsbedingungen von -20°C und 0.05 mbar entstanden optisch geeignete Lyophilisate. Das optimierte Lyophilisationsprogramm ist in Tabelle 18 zusammen gefasst.

Lyophilisation von DC30 Lipoplexe:

Die nach Beispiel 3 hergestellten und nach Beispiel 5 abgefüllten DC30 Lipoplexe wurden anschließend lyophilisiert. Als Lyophilisator wurde der Lyo Com 5018 der Firma Hof (Lohra, DE) verwendet. Die Gesamtzeit des Lyophilisationsprozesses betrug 64 Std. Die Vakuumregelung erfolgte über ein Vakuumventil. Als Vakuummesssonde wurde eine Sonde der Firma Pirani (Thyracont Elektronic GmbH, Passau, DE) eingesetzt. Die Trocknung erfolgte ohne Gefriertrocknungsbleche. Das Verschließen der Gefäße erfolgte bei einem Druck von 800 mbar. Das genaue Lyophilisationsprogramm ist in Tabelle 18 zusammen gefasst.

Tabelle 18:

| | Dauer (hh:mm) | Sol-Temperatur (°C) | Vakuum (mbar) |
|---|---|---|---|
| Beladen | 00:00 | 5 | 1000 |
| Einfrieren | 00:55 | -50 | 1000 |
| | 02:00 | -50 | 1000 |
| Haupttrocknung | 00:05 | -50 | 0,05 |
| | 01:30 | -20 | 0,05 |
| | 47:00 | -20 | 0,05 |
| Nachtrocknung | 02:00 | 30 | 0,05 |
| | 10:00 | 30 | 0,05 |
| Entnehmen | 00:30 | 5 | 0,05 |
| Gesamtzeit | 64:00 | ------- | |

**EP 1 512 393 A1**

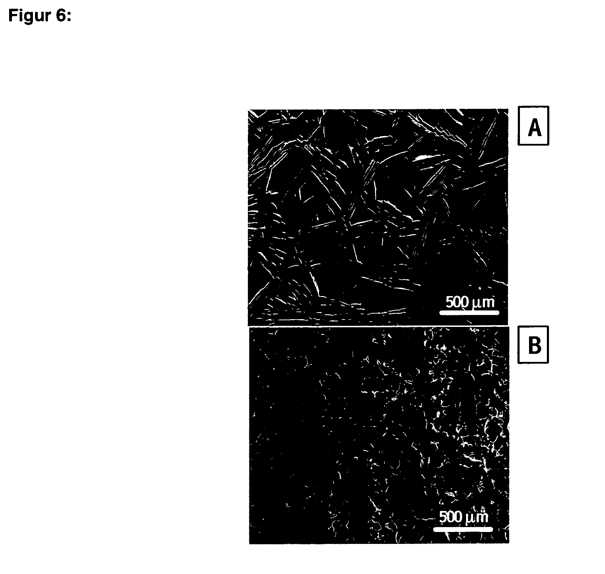

Das Endprodukt (Vial) wurde aus dem Lyophilisator entnommen und die Vial verbördelt. Die Lagerung erfolgte bei 2-8 °C. Der gebildete Lyophilisationskuchen war nicht kollabiert. In Figur 6A und 6B sind REM (Raster Elektronen Mikroskopie) Aufnahmen eines Lyophilisationskuchen gezeigt. Abbildung 6A zeigt die Oberflächenmorphologie des Lyophilisationskuchen. Abbildung 6B zeigt ein Ausschnitt im Lyophilisationskuchen selber. Diese Art von Lyophilisationskuchen haben sehr kurze Rekonstitutionszeiten von wenigen Sekunden.

Die Restfeuchte (mittels Karl Fischer Titration bestimmt) der DC30 Lipoplexe der Chargen aus Tabelle 9, sind in Tabelle 19 zusammengefasst. Es wurden jeweils 5 Vial pro Charge ausgewählt und deren Restfeuchte bestimmt.

Tabelle 19:

| Probe | Charge 1 (%) 1 Extrusion | Charge 2 (%) 2 Extrusionen | Charge 3 (%) 3 Extrusionen |
|---|---|---|---|
| 1 | 0.77 | 0.76 | 0.74 |
| 2 | 0.95 | 0.70 | 0.66 |
| 3 | 0.74 | 0.75 | 0.83 |
| 4 | 0.73 | 1.05 | 0.52 |
| 5 | 0.76 | 0.96 | 0.73 |
| **Mittelwert** | **0.79** | **0.84** | **0.70** |
| **SD** | 009 | 0.15 | 0.12 |
| **VK** | 11.5 | 18.15 | 16.58 |
| SD: Standardabweichung, VK: Variationskoeffizienz | | | |

Die Restfeuchte der Lyophilisate lag bei ≤ 3% . Es ist kein signifikanter Unterschied in der Restfeuchte zwischen den einzelnen Chargen zu erkennen. Es ist bekannt, dass die Restfeucht einen erheblichen Einfluss hat auf die Stabilität des Produktes.

**Beispiel 8:** Lyophilisation von homogenen Lipoplexen bestehend aus DOPE/DAC-Chol 70/30 (DAC30) und Nukleinsäure

Die Lyophilisation erfolgte in dem beschriebenen Beispiel in einem Lyophilisator: Lyo Epsilon 2-12D Fa.Christ (Osterode, DE). Die Vakuumregelung erfolgte mit einer Pirani Sonde. Die Lyophilisation erfolgte ohne Bleche. Genaue Angaben zum Lyophilisationprogramm sind in Tabelle 20 zusammengefasst.

Tabelle 20:

| Prozeßschritt | Zeit (hh: mm) | Temperatur (°C) | Druck (mbar) |
|---|---|---|---|
| Beladen | 00:00 | +5 | 1000 |
| Einfrieren | 00:55 | -50 | 1000 |
| Einfrieren | 02:00 | -50 | 1000 |
| Haupttrocknung | 00:05 | -50 | 0.05 |
| Haupttrocknung | 01:30 | -20 | 0.05 |
| Haupttrocknung | 47:00 | -20 | 0.05 |
| Nachtrocknung | 2:00 | +30 | 0.05 |
| Nachtrocknung | 10:00 | +30 | 0.05 |
| Entnehmen | 00:30 | +5 | 0.05 |
| **Gesamtzeit** | 64:00 | --- | --- |

Das Primärpackmittel entsprach dem in Beispiel 5. Die Daten der Restfeuchte (nach Karl Fischer bestimmt) von DAC30 Lipoplexe sind in Tabelle 21 (für n = 10 Vial) zusammengefasst. Der Wassergehalt von Lipoplexen ist ein wichtiger Faktor für die Langzeitstabilität des Produktes.

Tabelle 21:

| Probe | Wassergehalt in % |
|---|---|
| 1 | 0.65 |
| 2 | 0.54 |
| 3 | 0.71 |
| 4 | 0.65 |
| 5 | 0.78 |
| 6 | 0.65 |
| 7 | 0.71 |
| 8 | 1.29 |
| 9 | 0.85 |
| 10 | 0.90 |
| **Mittelwert** | **0.77** |

Die Ergebnisse der Restfeucht der DAC30 Lipoplexe lag wiederum unterhalb 1%. Nach Rekonstitution der Lyophilisate mit 1,5 ml WFI wurde der Durchmesser der DAC30 Lipoplexe bestimmt. In Tabelle 22 sind die Ergebnisse für 4 unabhängigen Chargen nach dem oben beschriebenen Herstellverfahren dargestellt. Es sind sowohl die Daten der Lipoplexgröße 30 min nach Herstellung (vor Lyo) und nach der Lyophilisation (nach Lyo) (vergleiche auch Tabelle 10) gezeigt.

Tabelle 22:

| Charge | Vor Lyophilisation | | Nach Lyophilisation | |
|---|---|---|---|---|
| | Größe (nm) | Poly-Index | Größe (nm) | Poly-Index |
| **1** | 309 | 0.27 | 313 | 0.29 |
| **2** | 300 | 0.28 | 303 | 0.32 |
| **3** | 312 | 0.24 | 310 | 0.25 |
| **4** | 320 | 0.23 | 320 | 0.26 |

Das verwendete Lyophilisationsprogram hat keinen destabilisierenden Einfluss auf die Lipoplexgröße.

Da der Gesamtprozess unter aseptischen Bedingungen durchgeführt wurde (werden kann), wurde eine Keimzahlbestimmung am DAC30 Lipoplex-Endprodukt durchgeführt. Die Keimzahlbestimmung erfolgte nach der Methode in des Europäischen und US-Arzneibuchs. In Tabelle 23 sind die Ergebnisse zusammengefasst für verschiedene DAC30 Lipoplexchargen.

Tabelle 23

| Lipoplexe nach Lyo Charge | Keimzahl (KBE) pro mL/ g | Keimzahl (KBE) pro mL/ g |
|---|---|---|
| | **Bakterien** | **Pilze** |
| **Charge 1** | < 1 / 2,5 Vials | < 1 / 2,5 Vials |
| **Charge 2** | < 1 / 2,5 Vials | < 1 / 2,5 Vials |
| **Charge 3** | < 1 / 2,5 Vials | < 1 / 2,5 Vials |
| **Charge 4** | < 1 / 2,5 Vials | < 1 / 2,5 Vials |
| KBE: Kolonie Bildende Einheit | | |

Beispiel 9: Bioaktivität und in *vitro* Transfektion von Lipoplexen

Einfluss der Mischungsreihenfolgg der Nukleinäurelösung und Liposomendispersion auf die Produktqualität:

Die Reihenfolge des Mischen der Nukleinäurelösung und der Liposomendispersion (Lipid zu Nukleinsäure = LzD, Nukleinsäure zu Lipid = DzL) (L: Lipid, D: DNA) hat sowohl einen Einfluss auf die Stabilität der Lipoplexe als auch auf deren Transfektionseigenschaft. Dies hängt unter anderem vom verwendeten Lipid, vom Lipid zu Nukleinsäure Verhältnis, Gesamtlipidkonzentration, und Formulierungspuffer ab.

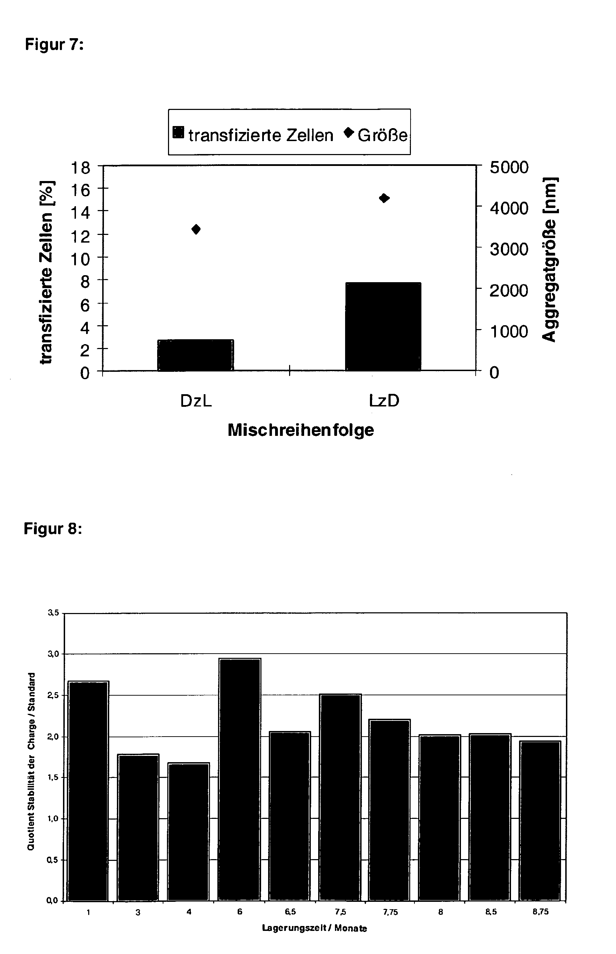

Figur 7 zeigt die Ergebnisse am Beispiel des kommerziell erhältlichen Lipids Lipofectin (Invitrogen life technologies, Carlsbad, USA). Wie aus Figur 7 hervorgeht, variierten sowohl die Lipoplexgrösse als auch die Transfektionseffizienz (durch Expression von EGFP bestimmt). Letztere wird sehr stark durch die Art und Weise des Zusammenführens und Mischens der beiden Lösungen beeinflusst. Durch LzD hergestellte Lipoplexe zeigten im Vergleich zu den DzL-Lipoplexen eine doppelt so hohe Transfektionseffizienz. Die Teilchengröße variierte ebenfalls (Figur 7) und hing von dem Mischungsprozess ab.
Ähnliche Versuche wurden auch mit dem Lipid DAC30 durchgeführt, und zusammenfassend kann man festhalten, dass die DzL-Lipoplexe etwas besser transfizieren als LzD-Lipoplexe. Beim Lipofectin führte die umgekehrte Mischungsreihenfolge zu besseren Ergebnissen. Beide Präparationsmethoden (DzL und LzD) neigten zur Aggregation und zur Trübung der Lipoplexlösung. Die so hergestellten Lipoplexe waren in einer Flüssigformulierung nicht stabil und eignen sich somit nicht zur Weiterverarbeitung (Abfüllung und anschließende Lyophilisation). Des weiteren lieferten diese zwei Verfahren (DzL und LzD) Lipoplexe mit nicht reproduzierbaren Teilchengrößen. Die Teilchengrößen variierten von Charge zu Charge um mehr als 300%.

Vorbehandlung der Liposomen auf die Lipoplex Transfektionseigenschaften:

Die Vorbehandlung der Liposomen, extrudiert gegenüber nicht-extrudiert, hat einen Einfluss auf die Transfektionseffizienz und die Teilchengröße der hergestellten Lipoplexe. Tabelle 24 zeigt die Transfektionseffizienz (ausgedrückt in % transfizierter Zellen) von DAC30/pAH7-EGFP 4:1 (w/w) Lipoplexen, die durch Komplexierung des Plasmids mit Liposomen durchgeführt wurde, die entweder nicht-extrudiert oder 1x durch eine 800 nm Extrusionsmembran extrudiert und über das Y-Stück gemischt wurden. Die Transfektionseffizienz für Lipoplexe die mit extrudierten Liposomen hergestellt wurden lag doppelt so hoch wie die für Lipoplexe aus nicht extrudierten Liposomen.

Tabelle 24:

| | Transfektionseffiziens / % | |
|---|---|---|
| | **nicht-extrudiert** | **extrudiert** |
| HA SMC | 3.7 | 7.9 |
| A-10 SMC | 12.0 | 26.4 |
| HA SMC = human aorta smooth muscle cell, A-10 SMC = rat smooth muscle cell | | |

Bioaktivität der Lipoplexchargen die nach Beispiel 4 hergestellt wurden:

Die Bioaktivität (Transfektionseigenschaft) von Lipoplexen bestehend aus DAC30 und pMCP-1 Plasmid im Massenverhältnis 5:1 wurde durch Transfektion von BHK21 Zellen getestet. Die Messung des exprimierten MCP-1 Proteins transfizierter Zellen wurde mittels eines BD OptEIA™ Human MCDA ELISA Kit BD Biosciences Pharmingen durchgeführt. Die Gesamtproteinbestimmung erfolgte mittels eines kommerziell erhältlichen Test (BCA, bicinchoninic acid).

$$\text{Potency} = \frac{\text{Konz. pMCP-1 in pg/mL}}{\text{Konz. Protein in } \mu\text{g/mL}}$$

Aus Tabelle 25 ist ersichtlich, dass die Bioaktivität der frisch hergestellten Lipoplexe (vor Lyo) und der lyophilisierten Lipoplexe (nach Lyo) vergleichbar war. Das Verhältnis der Potency vor/nach Lyophilisation beträgt ~1.10 und weist somit auf unveränderte Bioaktivität hin.

Tabelle 25:

| 6 well plate | Bioaktivität Lipoplex | |
| --- | --- | --- |
| | vor Lyophilisation | nach Lyophilisation |
| Charge | Potency MW (pg MCP-1 / μg Protein) | Potency MW (pg MCP-1 / μg Protein) |
| Nicht verdünnt | 1182 | 1072 |
| 1:2 verdünnt | 819 | 746 |
| 1:5 verdünnt | 312 | 314 |

Das Verhältnis von exprimierten Protein vor und nach Lyophilisation ist für vier unabhängige Chargen in Tabelle 26 zusammengefasst. Wiederum erkennt man, dass die Bioaktivität der Lipoplexe erhalten blieb.

Tabelle 26:

| Charge | Quotient Proteinexpression vor/nach Lyophilisation |
| --- | --- |
| 1 | 1.11 |
| 2 | 1.02 |
| 3 | 1.02 |
| 4 | 1.12 |

**Beispiel 10:** Langzeitstabilität von DAC30 Lipoplexen

Die Lagerungsstabilität der Lipoplexe DAC30/pMCP-1 5:1 (w/w) (als Lyophilisat) wurde bei verschiedenen Temperaturen bestimmt (siehe Figur 8). Es wurden folgende Parameter bestimmt: Größe der Lipoplexe, Homogenität (Polydispersitätsindex PI), DNA Gehalt, DNA Integrität der ccc-Form, Restfeuchte (als Quotient angegeben: Messwert/ Nullwert) und Bioaktivität (ausgedrückt als Transfektionseffizienz der Testcharge bezogen auf einen internen Standard). Die Lagerung dieser Lipoplexe bei 2-8 °C für 8 Monate (Tabelle 27) zeigt, dass die Größe der Lipoplexe im Bereich von 300 - 330 nm liegt mit PI < 0.3. Der DNA-Gehalt sowie die Integrität der DNA (ausgedrückt als % ccc-Form) veränderten sich im Rahmen der Messgenauigkeit kaum. Auch die Restfeucht veränderte sich nicht .

Tabelle 27:

| T (°C) | Ziehungs Zeitpunkt | Grösse (nm) | Poly-Index | Gehalt DNA (μg/ml) | DNA Integrität ccc-Form (%) | Restfeuchte verhältniss |
| --- | --- | --- | --- | --- | --- | --- |
| 4 °C | 0 Monate | 310 | 0,25 | 26 | 74 | 1 |
| | 4 Monate | 310 | 0,26 | 29 | 77 | 0,9 |
| | 8 Monate | 319 | 0,26 | 27 | 77 | 1,06 |
| 25 °C | 4 Monate | 328 | 0,25 | 25 | 77 | 1,1 |
| T = Lagertemperatur, Sollwert DNA Gehalt = 25 μg/ml, Restfeuchteverhältniss = Messwert/Nullwert | | | | | | |

In Figur 8 sind die Ergebnisse für die Bioaktivität der Lipoplexe (bei 4°C gelagert) über eine Periode von 8 Monaten zusammen getragen. Man erkennt, dass der Quotient der Bioaktivität der zu testenden Charge bezogen auf einen internen Standard Werte um 2 einnimmt.

Die Lagerung der flüssigen Lipoplexe bei 37 °C führt schon nach etwa 2 Monaten zu einer drastischen Reduzierung der Bioaktivität. Der Quotient beträgt nur noch 0.1.

Die Lagerung der Lipoplexe als Lyophilisat bei 37 °C zeigt, dass der Quotient nach 1 Monat bei 0.82 und nach 2.5 Monaten bei 0.5 lag. Die Lagerung der Lipoplexe (Lyophilisat) bei 25°C zeigt nach 4 Monaten noch einen Bioaktivitätsquotienten von über 2.

**Patentansprüche**

1. Liposomenmischung bestehend aus Liposomen mit einer definierten Größe zwischen 250 und 800 nm, wobei die Liposomen ein kationisches Lipid und eine neutrales Amphiphil enthalten *dadurch gekennzeichnet,* **dass** der Polydispersitätsindex der Liposomenmischung einen Wert $\leq$ 0.60 aufweist.

2. Liposomenmischung nach Anspruch 1 *dadurch gekennzeichnet,* **dass** es sich bei dem kationischen Lipid um DC-Chol ((3-beta[N(N',N'-Dimethylaminoethan) carbamoyl]cholesterin)) **oder** um DAC-Chol ((3-beta[N(N,N'-Dimethylaminoethan)carbamoyl]cholesterin)) handelt.

3. Liposomenmischung nach Anspruch 1 oder 2 *dadurch gekennzeichnet,* **dass** es sich bei dem neutralen Amphiphil um ein Cholinderivat (Dimyristoylphosphatidylcholin (DMPC), Dipalmitoylphosphatidylcholin (DPPC), Dioleoylphosphatidylcholin (DOPC)) **oder** um ein Ethanolaminderivat (Dimyristoylphosphatidylethanolamin (DMPE), Dipalmitoylphosphatidylethanolamin (DPPE), Dioleoylphosphatidylethanolamin (DOPE)) handelt.

4. Liposomenmischung nach Anspruch 3 *dadurch gekennzeichnet,* **dass** die Liposomen DOPE als neutrales Amphiphil und DC-Chol und/oder DAC-Chol als kationisches Lipid enthalten oder aus diesen bestehen, wobei das Massenverhältnis von DOPE zu dem kationischen Lipid 70:30 beträgt.

5. Verfahren zur Herstellung von homogenen Liposomen, bei dem eine Lipidsuspension in einem kontinuierlichen Verfahren durch eine poröse Membran extrudiert wird, *dadurch gekennzeichnet,* **dass** die Extrusion unter Niederdruckbedingungen bei kleiner $3\times10^5$ Pa durchgeführt wird.

6. Verfahren nach Anspruch 5 *dadurch gekennzeichnet,* **dass** die homogenen Liposomen ein kationisches Lipid und ein neutrales Amphiphil enthalten.

7. Verfahren nach Anspruch 6 *dadurch gekennzeichnet,* **dass** es sich bei dem kationischen Lipid um ein Cholesterol-Derivat handelt.

8. Verfahren nach Anspruch 7 *dadurch gekennzeichnet,* **dass** es sich bei dem Cholesterol-Derivat um DC-Chol ((3-beta[N(N',N'-Dimethylaminoethan) carbamoyl]cholesterin)) **oder** um DAC-Chol ((3-beta[N(N,N'-Dimethylaminoethan)carbamoyl]cholesterin)) handelt.

9. Verfahren nach Anspruch 8 *dadurch gekennzeichnet,* **dass** es sich bei dem neutralen Amphiphil um ein Cholinderivat (Dimyristoylphosphatidylcholin (DMPC), Dipalmitoylphosphatidylcholin (DPPC), Dioleoylphosphatidylcholin (DOPC)) **oder** um ein Ethanolaminderivat (Dimyristoylphosphatidylethanolamin (DMPE), Dipalmitoylphosphatidylethanolamin (DPPE), Dioleoylphosphatidylethanolamin (DOPE)) handelt.

10. Verfahren nach Anspruch 9 *dadurch gekennzeichnet,* **dass** die Liposomen DOPE als neutrales Amphiphil und DC-Chol und/oder DAC-Chol als kationisches Lipid enthalten oder aus diesen bestehen, wobei das Massenverhältnis von DOPE zu dem kationischen Lipid 70:30 beträgt.

11. Verfahren nach einem der Ansprüche 5 bis 10 *dadurch gekennzeichnet,* **dass** die Liposomenkonzentration in der Lipidsuspension 0.04 - 5 mg/ml, vorzugsweise 0.1 - 2 mg/ml, insbesondere 0.1 - 1 mg/ml beträgt.

12. Verfahren nach einem der Ansprüche 6 bis 11 *dadurch gekennzeichnet,* **dass** die Lipidsuspension eine Suspension eines kationischen Lipids und eines neutralen Amphiphil in einer wässrigen Lösung ist.

13. Verfahren nach Anspruch 12 bei dem die Suspension zusätzlich Salze, Polymere und/oder Zuckerverbindungen enthält.

14. Verfahren nach einem der Ansprüche 6 bis 13 *dadurch gekennzeichnet,* **dass** die Extrusion mit einer Flussrate zwischen 10 und 250 ml/min durchgeführt wird.

15. Verfahren nach einem der Ansprüche 6 bis 14 *dadurch gekennzeichnet,* **dass** die Extrusion bei Raumtemperatur durchgeführt wird.

16. Verfahren nach einem der Ansprüche 6 bis 15 *dadurch gekennzeichnet,* **dass** die Extrusion unter aseptischen Bedingungen in einem geschlossenen System durchgeführt wird.

17. Verfahren nach einem der Ansprüche 6 bis 16 ***dadurch gekennzeichnet,* dass** die poröse Membran eine Polycarbonatmembran ist.

18. Verfahren nach einem der Ansprüche 6 bis 17 ***dadurch gekennzeichnet,* dass** die poröse Membran Poren im Größenbereich von 600 nm bis 900 nm besitzt.

19. Verfahren nach einem der Ansprüche 6 bis 18 ***dadurch gekennzeichnet,* dass** die Lipidsuspension kontinuierlich zwischen 2 und 20 mal durch die poröse Membran extrudiert wird.

20. Liposomen erhältlich nach einem Verfahren gemäß der Ansprüche 18 oder 19.

21. Verfahren zur Herstellung von Lipoplexen ***dadurch gekennzeichnet,* dass** Liposomen nach Anspruch 20 mit Nukleinsäuremolekülen gemischt werden.

22. Verfahren nach Anspruch 21 ***dadurch gekennzeichnet,* dass** die Mischung von Liposomen und Nukleinsäuremolekülen über ein Y-Stück erfolgt, welches ein gleichmäßiges und kontinuierliches Zusammenführen der Liposomen und Nukleinsäuremoleküle (gleiche Volumina) erlaubt.

23. Verfahren nach Anspruch 21 oder 22 ***dadurch gekennzeichnet,* dass** die Konzentration der Liposomen bei der Mischung zwischen 0.02 und 1 mg/ml beträgt.

24. Verfahren nach einem der Ansprüche 21 bis 23 ***dadurch gekennzeichnet,* dass** das Liposomen-Nukleinsäure-Ladungsverhältnis (+/-) zwischen 4 und - 0.01 beträgt.

25. Verfahren nach Anspruch 24 ***dadurch gekennzeichnet,* dass** das Liposomen-Nukleinsäure-Ladungsverhältnis (+/-) zwischen 0.75 und 1.25 beträgt.

26. Verfahren nach einem der Ansprüche 21 bis 25 ***dadurch gekennzeichnet,* dass** Liposomen und Nukleinsäure mit einer Flussrate von 20 bis 800 ml/min gemischt werden.

27. Verfahren nach Anspruch 26 ***dadurch gekennzeichnet,* dass** Liposomen und Nukleinsäure mit einer Flussrate von 100 bis 500 ml/min gemischt werden.

28. Verfahren zur Herstellung von homogenen Lipoplexmischungen mit Lipoplexen mit einer Größe von 250 - 600 nm, vorzugsweise 275 - 500 nm, besonders bevorzugt 275 - 400 nm und einem Polydispersitätsindex von ≤ 0.5 enthaltend die Schritte:

   a) Extrusion einer Lipidsuspension, enthaltend DC30 oder vorzugsweise DAC30, in einem kontinuierlichen Verfahren durch eine 600 - 900 nm Membran, vorzugsweise mit einer Flussrate zwischen 10 - 250 ml/min, besonders bevorzugt zwischen 50 - 150 ml/min, weiter bevorzugt zwischen 75 - 120 ml/min, wobei die Lipidkonzentration in der Lipidsuspension vorzugsweise zwischen 0.04 - 5 mg/ml, bevorzugt zwischen 0.1 - 2 mg/ml, besonders bevorzugt zwischen 0.1 - 1 mg/ml, noch weiter bevorzugt zwischen 0.25 - 1 mg/ml beträgt, und die Lipidsuspension zumindest einmal, vorzugsweise aber kontinuierlich zwischen 2 und 20 mal durch die Membran extrudiert wird; **und**
   b) die entsprechend hergestellten Liposomenmischung mit Nukleinsäuremolekülen, die zuvor sterilfiltriert wurden, vorzugsweise durch einen 0,2 μm Filter, über ein Y-Stück mit einer kontinuierlichen Flussrate von 20 - 800 ml/min, vorzugsweise von 100 - 500 ml/min und einem Liposomen-Nukleinsäure-Ladungsverhältnis (+/-) von 4 - 0.01, vorzugsweise von 2 - 0.1, besonders bevorzugt von 1.5 - 0.5 und noch weiter bevorzugt von 1.25 - 0.75 gemischt werden.

29. Verfahren nach einem der Ansprüche 21 bis 28 ***dadurch gekennzeichnet,* dass** die Herstellung der Lipoplexe unter aseptischen Bedingungen durchgeführt wird.

30. Lipoplexmischung bestehend aus Lipoplexen mit einer definierten Größe zwischen 250 und 600 nm, wobei die Lipoplexe aus einer Mischung aus Liposomen gemäß einem der Ansprüche 1 bis 4 und Nukleinsäuremolekülen bestehen ***dadurch gekennzeichnet,* dass** der Polydispersitätsindex der Lipoplexmischung einen Wert ≤ 0.50 aufweist.

**31.** Lipoplexe erhältlich nach einem Verfahren gemäß der Ansprüche 21 bis 30.

**32.** Verfahren zur Lyophilisation von Lipoplexen gemäß Anspruch 31 in Gegenwart eines geeigneten Stabilisators enthaltend die Schritte

a) einfrieren der Lipoplexmischung auf eine Temperatur von ≤ - 50°C;
b) trocknen der Lipoplexmischung bei ungefähr - 20°C für zumindest 35 Stunden;
c) nachtrocknen der Lipoplexmischung bei ungefähr 20°C für zumindest 10 Stunden.

**33.** Verfahren zur Lyophilisation einer Lipoplexmischung in Gegenwart eines geeigneten Stabilisators enthaltend die Schritte

a) einfrieren der Lipoplexmischung auf eine Temperatur von ≤ - 50°C mit einer Temperaturerniedrigungsrate von ungefähr ≤ 1 °C/min;
b) inkubieren der Lipoplexmischung bei ≤ - 50°C für zumindest 2 Stunden;
c) erwärmen der Lipoplexmischung auf ungefähr - 20°C mit einer Erwärmungssrate von ungefähr ≤ 0.3°C/min;
d) trocknen der Lipoplexmischung bei ungefähr - 20°C für zumindest 35 Stunden;
e) erwärmen der Lipoplexmischung von ca. -20°C auf ca. 20°C mit einer Erwärmungsrate von ungefähr ≤ 0.44°C/min;
f) nachtrocknen der Lipoplexmischung bei ca. 20°C für zumindest 10 Stunden.

**34.** Verfahren nach Anspruch 32 oder 33 *dadurch gekennzeichnet,* **dass** die Trocknung bei einem Druck zwischen 0.025 und 0.05 mbar erfolgt.

**35.** Lipoplexlyophilisate erhältlich nach einem der Ansprüche 32 bis 34.

**36.** Verwendung von Lipoplexen nach Anspruch 31, Lipoplexmischungen nach Anspruch 30 oder Lipoplexlyophylisaten nach Anspruch 35 als oder zur Herstellung eines Arzneimittel(s) in der Gentherapie.

**Figur 1:**

**Figur 2:**

**Figur 3:**

**Figur 4:**

**Figur 5:**

**Figur 6:**

**Figur 7:**

**Figur 8:**

Figur 9:

α

Eintrittsröhren

Austrittsröhre

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | WO 02/072068 A (HEYES JAMES ANTHONY ;SPRINGER CAROLINE JOY (GB); CANCER RES CAMPAI) 19. September 2002 (2002-09-19) * Seite 125, Zeile 3 - Zeile 35 * * Seite 133 - Seite 134; Beispiele 93,94 * | 1-4 | A61K9/127 |
| X | WO 01/19400 A (SCHLETTER JENS ;CARDIOGENE GENTHERAPEUTISCHE S (DE)) 22. März 2001 (2001-03-22) * Seite 18, Zeile 7 - Zeile 15 * * Seite 21, Zeile 22 - Seite 23, Zeile 14 * | 1-4 | |
| X | AJMANI P S ET AL: "3BETA N-(N',N'-DIMETHYLAMINOETHANE)-CARBAMOYL CHOLESTEROL (DC-CHOL)-MEDIATED GENE DELIVERY TO PRIMARY RAT NEURONS: CHARACTERIZATION AND MECHANISM" NEUROCHEMICAL RESEARCH, PLENUM PRESS, NEW YORK, US, Bd. 24, Nr. 5, 1999, Seiten 699-703, XP008026856 ISSN: 0364-3190 * Seite 700, Zeile 28 - Zeile 46 * | 1-4 | |
| X | O ZELPHATI, C NGUYEN: "stable and monodisperse lipoplex formulations for gene delivery" GENE THERAPY, Bd. 5, 1998, Seiten 1272-1282, XP002270287 * Seite 1273, Spalte 2, Zeile 42 - Seite 1274, Spalte 1, Zeile 4 * | 1,3 | |

**RECHERCHIERTE SACHGEBIETE (Int.Cl.7)**

A61K

Der vorliegende Recherchenbericht wurde für alle Patentansprüche erstellt

| Recherchenort | Abschlußdatum der Recherche | Prüfer |
|---|---|---|
| Den Haag | 17. Februar 2004 | Muller, S |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
----------------------------------------------
& : Mitglied der gleichen Patentfamilie, übereinstimmendes
    Dokument

EPO FORM 1503 03.82 (P04C03)

**Europäisches**

**Patentamt**

## GEBÜHRENPFLICHTIGE PATENTANSPRÜCHE

Die vorliegende europäische Patentanmeldung enthielt bei ihrer Einreichung mehr als zehn Patentansprüche.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

See Sheet B

**Europäisches Patentamt**

## MANGELNDE EINHEITLICHKEIT DER ERFINDUNG ERGÄNZUNGSBLATT B

Nummer der Anmeldung

EP 03 01 9663

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-4 vollständig

   Liposomenmischung bestehend aus Liposomen, die ein kationisches Lipid und ein neutrales Amphiphil enthalten.
   - - -

2. Ansprüche: 5-20,29,36 vollständig

   Verfahren zur Herstellung von homogenen Liposomen durch Extrusion, die bei kleiner 3*100000 Pa durchgeführt wird.
   - - -

3. Ansprüche: 21-31,36 vollständig

   Verfahren zur Herstellung von Lipoplexen durch eine Mischung von Liposomen und Nukleinsäuremolekulen
   - - -

4. Ansprüche: 32-35 vollständig

   Verfahren zur Lyophilisation von Lipoplexen
   - - -

## ANHANG ZUM EUROPÄISCHEN RECHERCHENBERICHT
## ÜBER DIE EUROPÄISCHE PATENTANMELDUNG NR.

EP 03 01 9663

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-02-2004

| Im Recherchenbericht angeführtes Patentdokument | | Datum der Veröffentlichung | Mitglied(er) der Patentfamilie | | Datum der Veröffentlichung |
|---|---|---|---|---|---|
| WO 02072068 | A | 19-09-2002 | WO | 02072068 A2 | 19-09-2002 |
| WO 0119400 | A | 22-03-2001 | DE | 19944262 A1 | 29-03-2001 |
| | | | AU | 1638801 A | 17-04-2001 |
| | | | WO | 0119400 A2 | 22-03-2001 |
| | | | EP | 1216027 A2 | 26-06-2002 |

EPO FORM P0461

Für nähere Einzelheiten zu diesem Anhang : siehe Amtsblatt des Europäischen Patentamts, Nr.12/82